(19) Europäisches Patentamt
European Patent Office
Office européen des brevets

(11) EP 4 592 278 A1

(12) **EUROPEAN PATENT APPLICATION**
published in accordance with Art. 153(4) EPC

(43) Date of publication:
**30.07.2025 Bulletin 2025/31**

(21) Application number: **23868268.6**

(22) Date of filing: **21.09.2023**

(51) International Patent Classification (IPC):
***C07D 231/12*** (2006.01) ***A61K 31/415*** (2006.01)
***A61K 31/4196*** (2006.01) ***A61P 9/10*** (2006.01)
***A61P 17/02*** (2006.01) ***A61P 25/00*** (2006.01)
***A61P 25/02*** (2006.01) ***A61P 25/16*** (2006.01)
***A61P 25/28*** (2006.01) ***C07D 249/08*** (2006.01)
***C12N 15/12*** (2006.01)

(52) Cooperative Patent Classification (CPC):
**A61K 31/415; A61K 31/4196; A61P 9/10; A61P 17/02; A61P 25/00; A61P 25/02; A61P 25/16; A61P 25/28; C07D 231/12; C07D 249/08; C07K 14/435**

(86) International application number:
**PCT/JP2023/034339**

(87) International publication number:
**WO 2024/063147 (28.03.2024 Gazette 2024/13)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **21.09.2022 JP 2022150521**

(71) Applicants:
- **Shiratori Pharmaceutical Co., Ltd.**
  **Narashino-shi, Chiba 275-0016 (JP)**
- **Tohoku University**
  **Sendai-shi, Miyagi 980-8577 (JP)**

(72) Inventors:
- **YOSHINO, Hiroshi**
  **Narashino-shi, Chiba 275-0016 (JP)**
- **KOMODA, Taichi**
  **Narashino-shi, Chiba 275-0016 (JP)**
- **ITO, Masahiko**
  **Narashino-shi, Chiba 275-0016 (JP)**
- **FUKUNAGA, Koji**
  **Sendai-shi, Miyagi 980-8577 (JP)**
- **KAWAHATA, Ichiro**
  **Sendai-shi, Miyagi 980-8577 (JP)**

(74) Representative: **Mewburn Ellis LLP**
**Aurora Building**
**Counterslip**
**Bristol BS1 6BX (GB)**

Remarks:
The complete document including Reference Table(s) and the Sequence Listing(s) can be downloaded from the EPO website

(54) **TRIPHENYLAZOLE COMPOUND**

(57) The present invention provides a compound represented by formula (I) and useful as a medicament, or a pharmaceutically acceptable salt thereof. The present invention further provides a pharmaceutical composition for the treatment or prevention of synucleinopathy.

EP 4 592 278 A1

Fig. 2

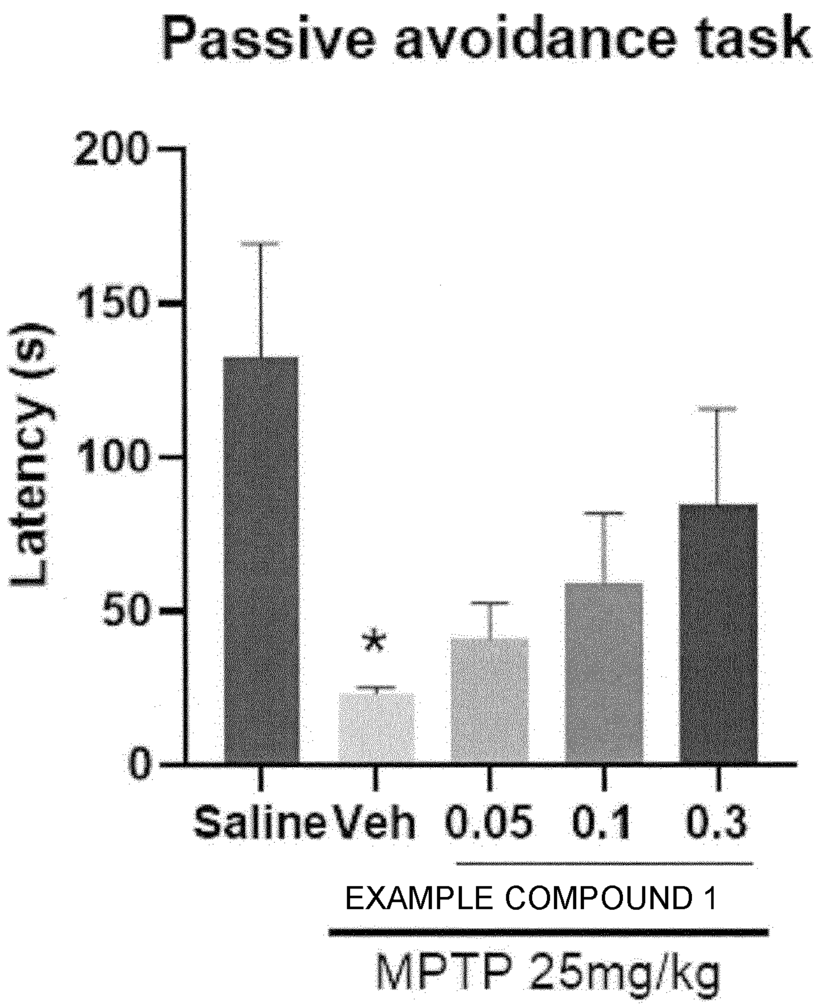


* $p < 0.05$ vs. Saline.

## Description

TECHNICAL FIELD

The present invention relates to a triphenylazole compound that binds to a fatty acid binding protein. Furthermore, the present invention relates to a pharmaceutical composition containing the compound, and a method of treatment or prevention using the compound.

BACKGROUND ART

α-Synuclein is a protein consisting of 140 amino acid residues encoded by the α-synuclein gene (SNCA) and is abundantly expressed at presynaptic terminals in the brain. The group of neurodegenerative diseases characterized by abnormal accumulation of α-synuclein is referred to as synucleinopathies, and accumulation in Lewy bodies and neurites in dementia with Lewy bodies (DLB), accumulation in glial cytoplasmic inclusions in multiple system atrophy, and the like are known. The involvement of polyunsaturated fatty acids in the oligomerization of α-synuclein has been reported (NPL 1). In Japan, which is facing a significant aging of its society, the number of patients with diseases that fall under the category of synucleinopathy has been increasing significantly in recent years.

Fatty acid binding proteins (FABPs) are cytoplasmic proteins with a molecular weight of 14 to 15 kDa and are expressed in a tissue-specific manner. FABPs have medium- to long-chain fatty acids as their ligands and are thought to be involved in lipid metabolism homeostasis and signal transduction. FABPs are known to have a plurality of subtypes having molecular structures similar to each other.

FABP3 is expressed in the brain, heart, skeletal muscle, mammary gland, and placenta (NPL 2). While its function is not fully understood, it is thought to be involved in lipid homeostasis, including lipid uptake and transport to the β-oxidation system in mitochondria, and to control the balance between neuronal excitation and suppression in the brain.

FABP3 has also been reported to promote α-synuclein aggregation (NPL 4 and 5).

α-Synuclein aggregates (inclusions) are expressed in substantia nigra dopamine neurons in Parkinson's disease (PD) and are diffusely expressed in the cerebral cortex in dementia with Lewy bodies (DLB). It has been reported that when fibrous synuclein is injected into the striatum of rats, aggregates propagate into the cerebral cortex in addition to the substantia nigra, forming synuclein inclusions in neurons (NPL 3).

FABP3 is highly expressed in dopaminergic neurons and promotes α-synuclein oligomerization and dopaminergic neuronal death after treatment with the dopaminergic neurotoxin 1-methyl-4-phenyl-1,2,3,6-tetrahydropyridine (MPTP) *in vivo* and *in vitro*. However, it has been reported that the administration of MPTP to FABP3-deficient mice does not produce neurotoxic effects (NPL 5), and that the compound Ligand 1 (also referred to as MF1), which is a FABP3 ligand with FABP3 inhibitory activity, protects dopaminergic neurons from cell death due to α-synuclein oligomerization (PTL 1 and NPL 6).

CITATION LIST

PATENT LITERATURE

PTL 1: WO2017/171053

NON PATENT LITERATURE

NPL 1: Sharon, R et al., Neuron, Vol. 37, 583-595, February 20, 2003

NPL 2: Yamamoto, Y. et al., J. Neurosci., 2018, 38(49):10411-10423

NPL 3: Paumier, K. L. et al., Neurobiology of Disease 2015; 82 :185-199

NPL 4: Misaki Onosato et al., 52nd Conference of the Pharmaceutical Society of Japan Tohoku Branch Abstracts 2013, vol.52, page 47

NPL 5: Shioda, N. et al., J. Biol. Chem., 289 (2014), pp. 18957-18965

NPL 6: Matsuo, K. et al., Neuropharmacology 150, (2019), 164-174


SUMMARY OF INVENTION

TECHNICAL PROBLEM

There is no established method of treatment or prevention that is sufficiently effective for neurodegenerative diseases classified as synucleinopathies, and there is a need for novel therapeutic and prophylactic agents.

In one aspect, an object of the present invention is to provide a pharmaceutical composition for use in the treatment or prevention of synucleinopathy. A further object of the present invention is to provide a method for treating or preventing synucleinopathy using a specific triphenylazole compound.

SOLUTION TO PROBLEM

The present inventors have conducted diligent research in order to achieve the above objects, and have found that when a halo C1-6 alkyl group such as a trifluoromethyl group is introduced, triphenylazole compounds have beneficial effects, such as suppressing α-synuclein aggregation, improving motor and cognitive dysfunction, and having neuroprotective effects, thereby accomplishing the present invention.

Furthermore, the present inventors have unexpectedly found that the triphenylazole compound of the present invention has a higher affinity for FABP3 than the known FABP3 ligand, compound Ligand 1 (PTL 1).

The disclosure of the present specification includes the inventions described in the following [1-1] to [1-31] and [2-1] to [2-31].

[1-1] A compound represented by formula (I):

[Formula 1]

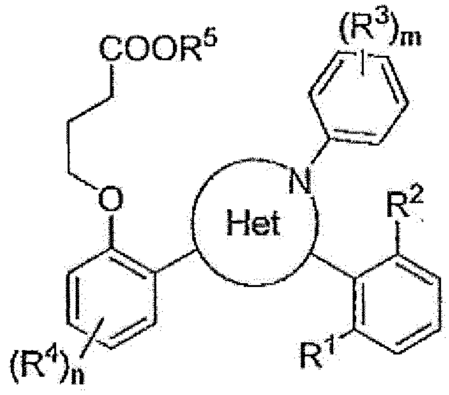

(I)

wherein $R^1$ is a hydrogen atom or a halo $C_{1-6}$ alkyl;
$R^2$ is a halo $C_{1-6}$ alkyl;
$R^3$ is selected from hydroxys, $C_{1-6}$ alkyls, $C_{1-6}$ alkoxys and halogen atoms;
$R^4$ is selected from halogens and $C_{1-6}$ alkyls;
$R^5$ is a hydrogen atom or a $C_{1-6}$ alkyl;
m is an integer selected from 0 to 3;

n is an integer selected from 0 to 3; and
Het is a 5-membered nitrogen-containing aromatic heterocyclic ring with one nitrogen atom and two carbon atoms substituted with phenyl groups,

or a pharmaceutically acceptable salt thereof.
[1-2] The compound or pharmaceutically acceptable salt thereof according to [1-1], wherein the compound is represented by formula (Ia):

[Formula 2]

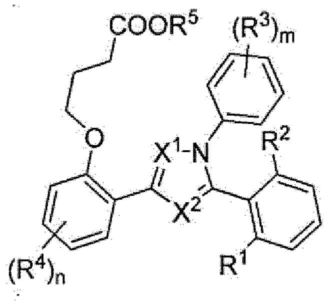

(I a)

wherein $R^1$, $R^2$, $R^3$, $R^4$, $R^5$, n, and m are as defined in [1] above;
$X^1$ and $X^2$ are independently a nitrogen atom or C-$R^{6a}$; and
$R^{6a}$ is a hydrogen atom, a halogen atom, or a $C_{1-6}$ alkyl.

[1-3] The compound or pharmaceutically acceptable salt thereof according to [1-1] or [1-2], wherein $X^1$ is a nitrogen atom and $X^2$ is CH, or $X^1$ and $X^2$ are nitrogen atoms.
[1-4] The compound or pharmaceutically acceptable salt thereof according to any of [1-1] to [1-3], wherein $R^2$ is trifluoromethyl.
[1-5] The compound or pharmaceutically acceptable salt thereof according to any of [1-1] to [1-4], wherein m is 0 or 1 and n is 0 or 1.
[1-6] The compound or pharmaceutically acceptable salt thereof according to any of [1-1] to [1-4], wherein m is 0 and n is 0 or 1.
[1-7] The compound or pharmaceutically acceptable salt thereof according to any of [1-1] to [1-6], wherein the compound is represented by formula (Ib):

[Formula 3]

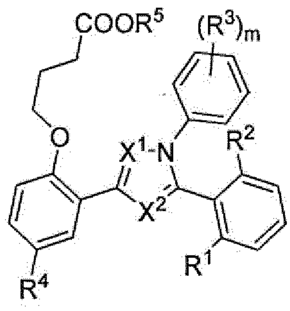

(I b)

wherein $R^1$, $R^2$, $R^3$, $R^4$, $R^5$, $X^1$, $X^2$, and m are as defined in any of [1-1] to [1-6].
[1-8] The compound or pharmaceutically acceptable salt thereof according to any of [1-1] to [1-7], wherein $R^1$ is a hydrogen atom.
[1-9] The compound or pharmaceutically acceptable salt thereof according to [1], wherein the compound is selected from 4-(4-fluoro-2-(1-phenyl-5-(2-(trifluoromethyl)phenyl)-1H-pyrazol-3-yl)phenoxy)butanoic acid;

4-(2-(1-phenyl-5-(2-(trifluoromethyl)phenyl)-1H-pyrazol-3-yl)phenoxy)butanoic acid;
4-(2-(1-phenyl-5-(2-(trifluoromethyl)phenyl)-1H-1,2,4-triazol-3-yl)phenoxy)butanoic acid;
4-(4-fluoro-2-(1-(4-isopropylphenyl)-5-(2-(trifluoromethyl)phenyl)-1H-pyrazol-3-yl)phenoxy)butanoic acid; and

4-(2-(1-(4-isopropylphenyl)-5-(2-(trifluoromethyl)phenyl)-1H-pyrazol-3-yl)phenoxy)butanoic acid.

[1-10] A pharmaceutical composition, containing the compound or pharmaceutically acceptable salt thereof according to any of [1-1] to [1-9].

[1-11] The pharmaceutical composition according to [1-10], for use in the treatment or prevention of synucleinopathy.

[1-12] The pharmaceutical composition according to [1-11], wherein the synucleinopathy is Parkinson's disease (PD), dementia with Lewy bodies (DLB), or multiple system atrophy.

[1-13] The pharmaceutical composition according to [1-10], for use in the treatment or prevention of a disease caused by dopamine neuronal dysfunction.

[1-14] The pharmaceutical composition according to [10], for use in the treatment or prevention of a cerebrovascular disorder such as cerebral infarction, cerebral hemorrhage, and subarachnoid hemorrhage, and head injury.

[1-15] The pharmaceutical composition according to [1-10], for use in the treatment or prevention of a psychiatric disorder such as autism and schizophrenia.

[1-16] The pharmaceutical composition according to any of [1-10] to [1-15], which is for oral administration.

[1-17] The compound or pharmaceutically acceptable salt thereof according to any of [1-1] to [1-9], for use in the treatment or prevention of synucleinopathy.

[1-18] The compound or pharmaceutically acceptable salt thereof according to [1-17], wherein the synucleinopathy is Parkinson's disease, dementia with Lewy bodies, or multiple system atrophy.

[1-19] The compound or pharmaceutically acceptable salt thereof according to any of [1-1] to [1-9], for use in the treatment or prevention of a disease caused by dopamine neuronal dysfunction.

[1-20] The compound or pharmaceutically acceptable salt thereof according to any of [1-1] to [1-9], for use in the treatment or prevention of a cerebrovascular disorder such as cerebral infarction, cerebral hemorrhage, and subarachnoid hemorrhage, and head injury.

[1-21] The compound or pharmaceutically acceptable salt thereof according to any of [1-1] to [1-9], for use in the treatment or prevention of a psychiatric disorder such as autism and schizophrenia.

[1-22] Use of the compound or pharmaceutically acceptable salt thereof according to any of [1] to [9], for the production of a pharmaceutical composition for the treatment or prevention of synucleinopathy.

[1-23] The use of the compound or pharmaceutically acceptable salt thereof according to [1-22], wherein the synucleinopathy is Parkinson's disease, dementia with Lewy bodies, or multiple system use of atrophy.

[1-24] Use of the compound or pharmaceutically acceptable salt thereof according to any of [1-1] to [1-9], for the production of a pharmaceutical composition for the treatment or prevention of a disease caused by dopamine neuronal dysfunction.

[1-25] Use of the compound or pharmaceutically acceptable salt thereof according to any of [1-1] to [1-9], for the production of a pharmaceutical composition for the treatment or prevention of a cerebrovascular disorder such as cerebral infarction, cerebral hemorrhage, and subarachnoid hemorrhage, and head injury.

[1-26] Use of the compound or pharmaceutically acceptable salt thereof according to any of [1-1] to [1-9], for the production of a pharmaceutical composition for the treatment or prevention of a psychiatric disorder such as autism and schizophrenia.

[1-27] A method for preventing or treating synucleinopathy, including administering to a subject an effective amount of the compound or pharmaceutically acceptable salt thereof according to any of [1-1] to [1-9].

[1-28] The prevention or treatment method according to [1-27], wherein the synucleinopathy is Parkinson's disease (PD), dementia with Lewy bodies (DLB), or multiple system atrophy.

[1-29] A method for preventing or treating a disease caused by dopamine neuronal dysfunction, including administering to a subject an effective amount of the compound or pharmaceutically acceptable salt thereof according to any of [1-1] to [1-9].

[1-30] A method for preventing or treating a cerebrovascular disorder such as cerebral infarction, cerebral hemorrhage, and subarachnoid hemorrhage, and head injury, including administering to a subject an effective amount of the compound or pharmaceutically acceptable salt thereof according to any of [1-1] to [1-9].

[1-31] A method for preventing or treating a psychiatric disorder such as autism and schizophrenia, including administering to a subject an effective amount of the compound or pharmaceutically acceptable salt thereof according to any of [1-1] to [1-9].

[2-1] A compound represented by formula (IX):

[Formula 4]

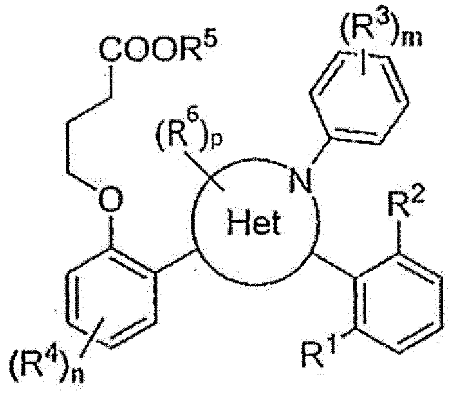

(I X)

wherein $R^1$ is a hydrogen atom or a $C_{1-6}$ haloalkyl;
$R^2$ is a $C_{1-6}$ haloalkyl;
$R^3$ is selected from hydroxys, $C_{1-6}$ alkyls, $C_{1-6}$ alkoxys and halogen atoms;
$R^4$ is selected from halogen atoms and $C_{1-6}$ alkyls;
$R^5$ is a hydrogen atom or a $C_{1-6}$ alkyl;
$R^6$ is a halogen atom or a $C_{1-6}$ alkyl;
m is an integer selected from 0 to 3;
n is an integer selected from 0 to 3;
p is an integer selected from 0 to 2; and
Het is a 5-membered nitrogen-containing aromatic heterocyclic ring with one nitrogen atom and two carbon atoms substituted with phenyl groups,

or a pharmaceutically acceptable salt thereof.
[2-2] The compound or pharmaceutically acceptable salt thereof according to [2-1], wherein the compound is represented by formula (Ia):

[Formula 5]

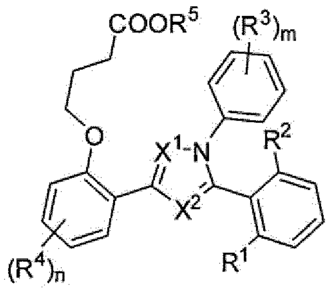

(I a)

wherein $R^1$, $R^2$, $R^3$, $R^4$, $R^5$, n, and m are as defined in [2-1] above;
$X^1$ and $X^2$ are independently a nitrogen atom or C-$R^{6a}$; and
$R^{6a}$ is a hydrogen atom, a halogen atom, or a $C_{1-6}$ alkyl.

[2-3] The compound or pharmaceutically acceptable salt thereof according to [2-1] or [2-2], wherein $X^1$ is a nitrogen atom and $X^2$ is CH, or $X^1$ and $X^2$ are nitrogen atoms.
[2-4] The compound or pharmaceutically acceptable salt thereof according to any of [2-1] to [2-3], wherein $R^2$ is trifluoromethyl.
[2-5] The compound or pharmaceutically acceptable salt thereof according to any of [2-1] to [2-4], wherein m is 0 or 1 and n is 0 or 1.
[2-6] The compound or pharmaceutically acceptable salt thereof according to any of [2-1] to [2-4], wherein m is 0 and n is 0 or 1.
[2-7] The compound or pharmaceutically acceptable salt thereof according to any of [2-1] to [2-6], wherein the

compound is represented by formula (Ib):

[Formula 6]

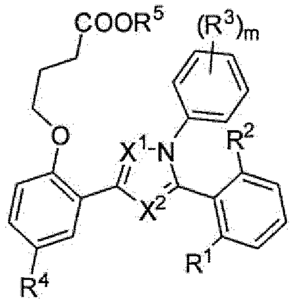

(I b)

wherein $R^1$, $R^2$, $R^3$, $R^4$, $R^5$, $X^1$, $X^2$, and m are as defined in any of [2-1] to [2-6].
[2-8] The compound or pharmaceutically acceptable salt thereof according to any of [2-1] to [2-7], wherein $R^1$ is a hydrogen atom.
[2-9] The compound or pharmaceutically acceptable salt thereof according to [2-1], wherein the compound is selected from 4-(4-fluoro-2-(1-phenyl-5-(2-(trifluoromethyl)phenyl)-1H-pyrazol-3-yl)phenoxy)butanoic acid;

4-(2-(1-phenyl-5-(2-(trifluoromethyl)phenyl)-1H-pyrazol-3-yl)phenoxy)butanoic acid;
4-(2-(1-phenyl-5-(2-(trifluoromethyl)phenyl)-1H-1,2,4-triazol-3-yl)phenoxy)butanoic acid;
4-(4-fluoro-2-(1-(4-isopropylphenyl)-5-(2-(trifluoromethyl)phenyl)-1H-pyrazol-3-yl)phenoxy)butanoic acid; and
4-(2-(1-(4-isopropylphenyl)-5-(2-(trifluoromethyl)phenyl)-1H-pyrazol-3-yl)phenoxy)butanoic acid.

[2-10] A pharmaceutical composition, containing the compound or pharmaceutically acceptable salt thereof according to any of [2-1] to [2-9].
[2-11] The pharmaceutical composition according to [2-10], for use in the treatment or prevention of synucleinopathy.
[2-12] The pharmaceutical composition according to [2-11], wherein the synucleinopathy is Parkinson's disease (PD), dementia with Lewy bodies (DLB), or multiple system atrophy.
[2-13] The pharmaceutical composition according to [2-10], for use in the treatment or prevention of a disease caused by dopamine neuronal dysfunction.
[2-14] The pharmaceutical composition according to [2-10], for use in the treatment or prevention of a cerebrovascular disorder such as cerebral infarction, cerebral hemorrhage, and subarachnoid hemorrhage, and head injury.
[2-15] The pharmaceutical composition according to [2-10], for use in the treatment or prevention of a psychiatric disorder such as autism and schizophrenia.
[2-16] The pharmaceutical composition according to any of [2-10] to [2-15], which is for oral administration.
[2-17] The compound or pharmaceutically acceptable salt thereof according to any of [2-1] to [2-9], for use in the treatment or prevention of synucleinopathy.
[2-18] The compound or pharmaceutically acceptable salt thereof according to [2-17], wherein the synucleinopathy is Parkinson's disease, dementia with Lewy bodies, or multiple system atrophy.
[2-19] The compound or pharmaceutically acceptable salt thereof according to any of [2-1] to [2-9], for use in the treatment or prevention of a disease caused by dopamine neuronal dysfunction.
[2-20] The compound or pharmaceutically acceptable salt thereof according to any of [2-1] to [2-9], for use in the treatment or prevention of a cerebrovascular disorder such as cerebral infarction, cerebral hemorrhage, and subarachnoid hemorrhage, and head injury.
[2-21] The compound or pharmaceutically acceptable salt thereof according to any of [2-1] to [2-9], for use in the treatment or prevention of a psychiatric disorder such as autism and schizophrenia.
[2-22] Use of the compound or pharmaceutically acceptable salt thereof according to any of [2-1] to [2-9], for the production of a pharmaceutical composition for the treatment or prevention of synucleinopathy.
[2-23] The use of the compound or pharmaceutically acceptable salt thereof according to [2-22], wherein the synucleinopathy is Parkinson's disease, dementia with Lewy bodies, or multiple system use of atrophy.
[2-24] Use of the compound or pharmaceutically acceptable salt thereof according to any of [2-1] to [2-9], for the production of a pharmaceutical composition for the treatment or prevention of a disease caused by dopamine neuronal dysfunction.
[2-25] Use of the compound or pharmaceutically acceptable salt thereof according to any of [2-1] to [2-9], for the production of a pharmaceutical composition for the treatment or prevention of a cerebrovascular disorder such as

cerebral infarction, cerebral hemorrhage, and subarachnoid hemorrhage, and head injury.

[2-26] Use of the compound or pharmaceutically acceptable salt thereof according to any of [2-1] to [2-9], for the production of a pharmaceutical composition for the treatment or prevention of a psychiatric disorder such as autism and schizophrenia.

[2-27] A method for preventing or treating synucleinopathy, including administering to a subject an effective amount of the compound or pharmaceutically acceptable salt thereof according to any of [2-1] to [2-9].

[2-28] The prevention or treatment method according to [2-27], wherein the synucleinopathy is Parkinson's disease (PD), dementia with Lewy bodies (DLB), or multiple system atrophy.

[2-29] A method for preventing or treating a disease caused by dopamine neuronal dysfunction, including administering to a subject an effective amount of the compound or pharmaceutically acceptable salt thereof according to any of [2-1] to [2-9].

[2-30] A method for preventing or treating a cerebrovascular disorder such as cerebral infarction, cerebral hemorrhage, and subarachnoid hemorrhage, and head injury, including administering to a subject an effective amount of the compound or pharmaceutically acceptable salt thereof according to any of [2-1] to [2-9].

[2-31] A method for preventing or treating a psychiatric disorder such as autism and schizophrenia, including administering to a subject an effective amount of the compound or pharmaceutically acceptable salt thereof according to any of [2-1] to [2-9].

ADVANTAGEOUS EFFECTS OF INVENTION

In one aspect, the present invention provides a compound with binding activity to fatty acid binding proteins, particularly FABP3. In another aspect, the present invention provides a compound with inhibitory activity against fatty acid binding proteins, particularly FABP3. In still another aspect, the present invention provides a compound with a therapeutic or prophylactic effect on synucleinopathy. In yet another aspect, the present invention provides a pharmaceutical composition for the treatment or prevention of synucleinopathy.

BRIEF DESCRIPTION OF DRAWINGS

[Fig. 1] Fig. 1 is a figure showing the nucleotide sequences of GST-FABP3, GST-FABP4, GST-FABP5, and GST-FABP7 that were sequenced in Test Example 1.

[Fig. 2] Fig. 2 is a graph showing the results of the cognitive function evaluation by the passive avoidance task in Test Example 2.

[Fig. 3] Fig. 3 is graphs showing the results of the cognitive function evaluation by the novel object recognition task in Test Example 3.

[Fig. 4] Fig. 4 is a graph showing the results of the motor function evaluation by the rotarod task in Test Example 4.

[Fig. 5] Fig. 5 is a graph showing the results of the motor function evaluation by the beam walking task in Test Example 4.

[Fig. 6] Fig. 6 is a graph showing the number of neurons expressing tyrosine hydroxylase (TH) in the brain sections containing the substantia nigra region prepared in Test Example 5.

[Fig. 7] Fig. 7 is photographs of brain sections containing the substantia nigra region prepared in Test Example 5 after staining for tyrosine hydroxylase-positive cells.

[Fig. 8] Fig. 8 is a graph showing the number of neurons expressing tyrosine hydroxylase (TH) in brain sections containing the ventrotegmental area (VTA) prepared in Test Example 5.

[Fig. 9] Fig. 9 is photographs of brain sections containing the ventrotegmental area (VTA) prepared in Test Example 5 after staining for tyrosine hydroxylase-positive cells.

[Fig. 10] Fig. 10 is photographs of brain sections containing the substantia nigra (SNpc) prepared in Test Example 5 after staining for phosphorylated $\alpha$-synuclein.

[Fig. 11] Fig. 11 is a graph showing that Example Compound 2 protected dopaminergic neurons from the toxicity of phosphorylated $\alpha$-synuclein in brain sections of the substantia nigra (SNpc) prepared in Test Example 5.

[Fig. 12] Fig. 12 is photographs of brain sections containing the ventrotegmental area (VTA) prepared in Test Example 5 after staining for phosphorylated $\alpha$-synuclein.

[Fig. 13] Fig. 13 is a graph showing that Example Compound 2 protected dopaminergic neurons from the toxicity of phosphorylated $\alpha$-synuclein in brain sections of the ventrotegmental area (VTA) prepared in Test Example 5.

[Fig. 14] Fig. 14 is photographs of brain sections containing the substantia nigra (SNpc) prepared in Test Example 5 after double staining for phosphorylated $\alpha$-synuclein and FABP3.

[Fig. 15] Fig. 15 is a graph showing that the interaction between phosphorylated $\alpha$-synuclein and FABP3 is inhibited by

Example Compound 2 in dopaminergic neurons in brain sections of the substantia nigra (SNpc) prepared in Test Example 5.

[Fig. 16] Fig. 16 is photographs of brain sections containing the ventrotegmental area (VTA) prepared in Test Example 5 after double staining for phosphorylated $\alpha$-synuclein and FABP3.

[Fig. 17] Fig. 17 is a graph showing that the interaction between phosphorylated $\alpha$-synuclein and FABP3 is inhibited by Example Compound 2 in dopaminergic neurons in brain sections of the ventrotegmental area (VTA) prepared in Test Example 5.

[Fig. 18] Fig. 18 is photographs of brain sections stained 24 hours after administration of various doses of Example Compound 1 at 30 minutes after reperfusion in the focal cerebral ischemia model prepared in Test Example 6.

[Fig. 19] Fig. 19 is a graph showing the infarct volume of the brain sections 24 hours after administration of various doses of Example Compound 1 at 30 minutes after reperfusion in the focal cerebral ischemia model prepared in Test Example 6, and a graph showing the neurological scores.

[Fig. 20] Fig. 20 is photographs of stained brain sections when Example Compound 1 is administered at 0.5, 1, and 2 hours after reperfusion in the focal cerebral ischemia model prepared in Test Example 6.

[Fig. 21] Fig. 21 is a graph showing the infarct volume of stained brain sections when Example Compound 1 is administered at 0.5, 1, and 2 hours after reperfusion in the focal cerebral ischemia model prepared in Test Example 6, and a graph showing the neurological scores.

[Fig. 22] Fig. 22 is photographs of brain sections stained 7 days after administration of various doses of Example Compound 1 at 30 minutes after reperfusion in the focal cerebral ischemia model prepared in Test Example 6.

[Fig. 23] Fig. 23 is a graph showing the infarct volume of the brain sections 7 days after administration of various doses of Example Compound 1 at 30 minutes after reperfusion in the focal cerebral ischemia model prepared in Test Example 6, and a graph showing the neurological scores.

[Fig. 24] Fig. 24 is a graph showing the mouse survival rates for Figs. 18 to 23.

[Fig. 25] Fig. 25 is a graph showing the mechanism of action of Example Compound 2 in dopaminergic neurons of PD model mice produced by MPTP treatment (Reference for Fig. 25: Fig. 6 in Journal of Pharmacological Sciences 152 (2023) 30-38).

DESCRIPTION OF EMBODIMENTS

The following describes more specifically the present invention.

One aspect of the present invention provides a pharmaceutical composition for the treatment or prevention of synucleinopathy or a disorder, which contains as an active ingredient a compound represented by formula (I) or formula (IX), or a pharmaceutically acceptable salt thereof. In one embodiment, a pharmaceutical composition for the treatment or prevention of synucleinopathy or a disorder, which contains as an active ingredient a compound represented by formula (Ia) or formula (Ib), is provided. In the present specification, the compound represented by formula (I) or formula (IX) includes the compound represented by formula (Ia) or formula (Ib).

As used herein, "$C_{1-6}$ alkyl" means a linear, branched, cyclic or partially cyclic alkyl group having 1 to 6 carbon atoms, and examples thereof include methyl, ethyl, n-propyl, isopropyl, n-butyl, sec-butyl, isobutyl, tert-butyl, n-pentyl, 3-methylbutyl, 2-methylbutyl, 1-methylbutyl, 1-ethylpropyl, n-hexyl, 4-methylpentyl, 3-methylpentyl, 2-methylpentyl, 1-methylpentyl, 3-ethylbutyl, 2-ethylbutyl, cyclopropyl, cyclobutyl, cyclopentyl, cyclohexyl, and cyclopropylmethyl, as well as $C_{1-4}$ alkyl and $C_{1-3}$ alkyl.

As used herein, "$C_{1-6}$ alkoxy" means an alkyloxy group [-O-($C_{1-6}$ alkyl)] with an alkyl group having 1 to 6 carbon atoms as already defined as the alkyl moiety, and examples thereof include methoxy, ethoxy, n-propoxy, isopropoxy, n-butoxy, sec-butoxy, isobutoxy, tert-butoxy, n-pentoxy, 3-methylbutoxy, 2-methylbutoxy, 1-methylbutoxy, 1-ethylpropoxy, n-hexyloxy, 4-methylpentoxy, 3-methylpentoxy, 2-methylpentoxy, 1-methylpentoxy, 3-ethylbutoxy, cyclopentyloxy, cyclohexyloxy, and cyclopropylmethyloxy, as well as $C_{1-4}$ alkoxy and $C_{1-3}$ alkoxy.

Examples of the halogen atom include a fluorine atom (F), a chlorine atom (Cl), a bromine atom (Br), and an iodine atom (I).

As used herein, "halo $C_{1-6}$ alkyl" means an alkyl substituted with one or more halogen atoms, and examples thereof include fluoromethyl, difluoromethyl, trifluoromethyl, chloromethyl, dichloromethyl, trichloromethyl, and methyl iodide.

When m is 0 in formula (I), (Ia), (Ib) or (IX), the substituent indicated by $R^3$ is absent. When m is 2 or more, the substituents indicated by $R^3$ may be the same or different. When n is 0 in formula (I), (Ia), (Ib) or (IX), the substituent indicated by $R^4$ is absent. When n is 2 or more, the substituents indicated by $R^4$ may be the same or different. When p is 0 in formula (IX), the substituent indicated by $R^6$ is absent. When p is 2, the substituents indicated by $R^6$ may be the same or different.

In the present specification, Het in formula (I) or formula (IX) represents a 5-membered nitrogen-containing aromatic heterocyclic ring having at least one nitrogen atom. Here, one nitrogen atom included as a ring atom in Het and

two carbon atoms are substituted with phenyl groups, and each phenyl group may have a substituent as shown in formula (I) or formula (IX). Examples of the 5-membered nitrogen-containing aromatic heterocyclic ring, which is a 5-membered nitrogen-containing aromatic heterocyclic ring, include pyrrole, imidazole, pyrazole, and triazole. In the present specification, the compound represented by formula (Ia) includes the compounds represented by the following formulas (Ia-1), (Ia-2), (Ia-3), and (Ia-4), or the pharmaceutically acceptable salts thereof.

[Formula 7]

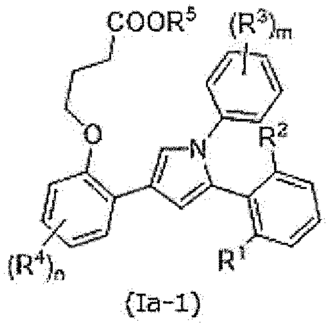
(Ia-1)

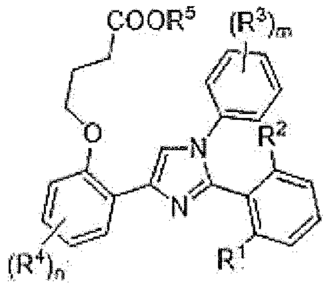
(Ia-2)

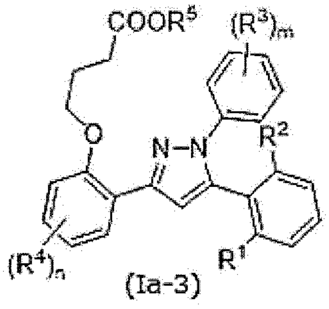
(Ia-3)

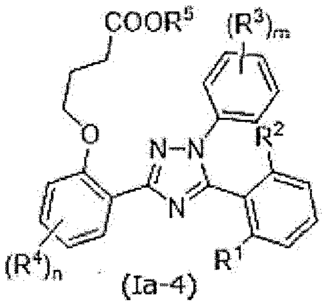
(Ia-4)

In the present specification, the compound represented by formula (Ib) includes the compounds represented by the following formulas (Ib-1), (Ib-2), (Ib-3), and (Ib-4), or the pharmaceutically acceptable salts thereof.

[Formula 8]

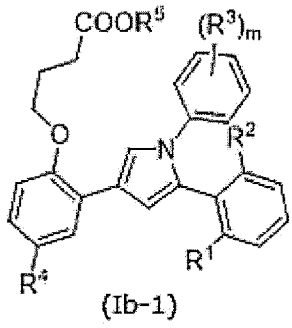
(Ib-1)

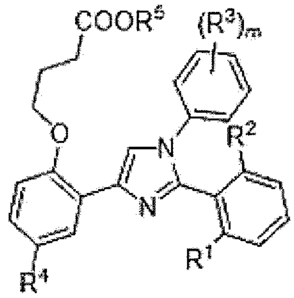
(Ib-2)

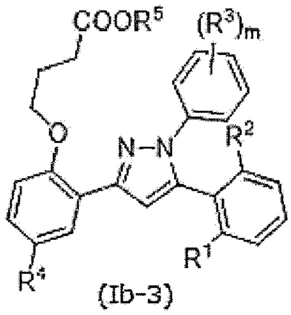
(Ib-3)

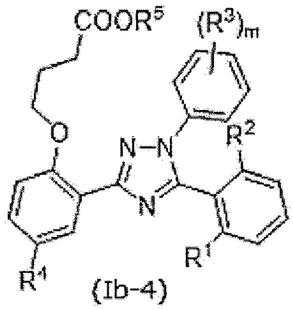
(Ib-4)

When the compound of formula (I) or formula (IX) or a pharmaceutically acceptable salt thereof forms a solvate, such as a hydrate, the present invention can be carried out using the solvate. Furthermore, the compound of the present invention or the pharmaceutically acceptable salt thereof can be used as a mixture, solution, crystalline polymorphs, or the like as appropriate to carry out the present invention.

As the compound of the present invention, for example, the compounds described in the Examples of the present specification can be used, and more specifically, the following compounds or pharmaceutically acceptable salts thereof can be used:

4-(4-fluoro-2-(1-phenyl-5-(2-(trifluoromethyl)phenyl)-1H-pyrazol-3-yl)phenoxy)butanoic acid;
4-(2-(1-phenyl-5-(2-(trifluoromethyl)phenyl)-1H-pyrazol-3-yl)phenoxy)butanoic acid;
4-(2-(1-phenyl-5-(2-(trifluoromethyl)phenyl)-1H-1,2,4-triazol-3-yl)phenoxy)butanoic acid;
4-(4-fluoro-2-(1-(4-isopropylphenyl)-5-(2-(trifluoromethyl)phenyl)-1H-pyrazol-3-yl)phenoxy)butanoic acid; and

4-(2-(1-(4-isopropylphenyl)-5-(2-(trifluoromethyl)phenyl)-1H-pyrazol-3-yl)phenoxy)butanoic acid.

The "pharmaceutically acceptable salt" of the compound of formula (I) or formula (IX) is not particularly limited as long as it is a salt that can be used as a medicament. Examples of the salt formed by the compound of the present invention with a base include salts with inorganic bases such as sodium, potassium, magnesium, calcium, and aluminum; and salts with organic bases such as methylamine, ethylamine, and ethanolamine.

In one embodiment of the present invention, the compound of formula (I) or formula (IX), an enantiomer, diastereomer, or pharmaceutically acceptable salt thereof is administered as a prodrug and converted to an active compound in the body.

Synucleinopathy in the present invention refers to neurodegenerative diseases characterized by abnormal accumulation of α-synuclein, such as PD, DLB, or multiple system atrophy, and the pharmaceutical composition and method of the present invention can be used, for example, to suppress the progression of synucleinopathy.

In the pharmaceutical composition and method of the present invention, the stage of progression, severity and pathology of the synucleinopathy to be treated is not particularly limited. Yahr I to V are used to classify the severity of PD. DLB has early, middle, and late stages, and can be divided into brainstem-predominant, limbic, and neocortical types based on the site of expression of α-synuclein aggregates. Furthermore, the modified Ranking Scale 1 to 6 is used to classify the severity of multiple system atrophy, which is classified according to the pathology, such as Shy Drager syndrome, olivopontocerebellar atrophy, and striatonigral degeneration. All are groups of diseases in which α-synuclein aggregates are expressed in neurons or glial cells, and the pharmaceutical composition and method of the present invention can be used for the treatment or prevention of a disease selected from these groups of diseases.

The onset of dementia is thought to be caused by neuronal cell death in the frontal cortex or hippocampus. In the present invention, it is proposed that the FABP3 ligand suppresses α-synuclein aggregation. As a result, neuronal cell death caused by α-synuclein aggregates in the brain is suppressed.

The pharmaceutical composition and method of the present invention can be used for the treatment and prevention of PD and DLB in particular.

In addition, the pharmaceutical composition and method of the present invention can improve and suppress the progression of motor dysfunction, cognitive dysfunction, and psychiatric disorders such as autism and schizophrenia.

Examples of motor dysfunction include tremor (shaking of hands and feet), akinesia (slowness of movement), rigidity (stiffness of muscles and resistance to bending and stretching of joints), and impairment of postural reflexes (difficulty in balancing the body) in PD, parkinsonian symptoms in DLB, and autonomic dysfunction (e.g., urinary dysfunction, erectile dysfunction, orthostatic hypotension, and decreased sweating), and cerebellar ataxia (ataxic gait and dysarthria, limb ataxia, or cerebellar eye movement disorder) in addition to parkinsonian symptoms in multiple system atrophy.

Examples of cognitive dysfunction include memory impairment, frontal and parietal lobe dysfunction (e.g., attentional, visuospatial, constructional, and executive function deficits), fluctuating cognition, and visual hallucinations in DLB. In PD, higher brain dysfunction in executive functions (planning, set shifting and maintenance, problem solving, etc.), memory functions such as working memory and procedural memory, and visuospatial functions may be seen in the early stages, and as it progresses, symptoms similar to DLB, such as visual hallucinations, visuospatial dysfunction, and slowed thinking may be seen. In multiple system atrophy, for example, dementia such as memory loss may be seen.

In addition, the pharmaceutical composition and method of the present invention can be used for the treatment and prevention of neurodegenerative diseases.

Examples of neurodegenerative diseases include amyotrophic lateral sclerosis (ALS), PD, Alzheimer's dementia, DLB, Guillain-Barre syndrome, chronic inflammatory demyelinating polyneuropathy, multiple sclerosis, and neuromyelitis optica.

In addition, the pharmaceutical composition and method of the present invention has a neuroprotective effect, and can therefore be used for the prevention and treatment of cerebrovascular disorders such as cerebral infarction, cerebral hemorrhage, and subarachnoid hemorrhage, and head injury.

Furthermore, it can be used for the prevention and treatment of dopamine-related diseases.

The pharmaceutical composition of the present invention can be made in various dosage forms, including, but not limited to, tablets, capsules, powders, granules, pills, liquids, emulsions, suspensions, solutions, spirits, syrups, extracts, and elixirs for oral administration, and as parenteral agents, injections such as subcutaneous, intravenous, intramuscular, and intraperitoneal injections; transdermal administration or patches, ointments or lotions; sublingual formulations or mucoadhesive patches for buccal administration; and aerosol formulations for nasal administration. These formulations can be produced by a known method commonly used in formulation processes.

The pharmaceutical composition can contain various generally used components, such as one or more pharmaceutically acceptable excipients, disintegrants, diluents, lubricants, flavoring agents, coloring agents, sweeteners, flavoring agents, suspending agents, wetting agents, emulsifiers, dispersants, adjuvants, preservatives, buffers, binders, stabilizers, and coating agents. In addition, the pharmaceutical composition of the present invention may be in a long-acting or sustained-release dosage form.

The dosage of the pharmaceutical composition of the present invention can be appropriately selected according to the route of administration, the patient's body type, age, and physical condition, the degree of disease, the time elapsed from onset, and the like, and the pharmaceutical composition of the present invention can contain the compound of formula (I) or formula (IX) above in effective amounts for treatment and/or prevention. In the present invention, the compound of formula (I) or formula (IX) above can generally be used in doses of 1 $\mu$g to 1,000 mg/day/adult, such as 1 $\mu$g to 200 mg/day/adult, specifically 5 $\mu$g to 100 mg/day/adult, and more specifically 10 $\mu$g to 50 mg/day/adult. The pharmaceutical composition may be administered in a single or plurality of doses.

The pharmaceutical composition of the present invention may contain, as necessary, conventionally known components such as coloring agents, preservatives, fragrances, flavoring agents, coating agents, antioxidants, vitamins, amino acids, peptides, proteins, and minerals (such as iron, zinc, magnesium, and iodine). The pharmaceutical composition of the present invention may be prepared in a form suitable for oral administration, such as in the form of various solid preparations such as granules (including dry syrups), capsules (soft capsules and hard capsules), tablets (including chewable tablets), powders (powders), and pills, or liquid preparations such as oral liquids (including liquids, suspensions, and syrups).

Examples of additives for formulation include excipients, lubricants, binders, disintegrants, fluidizers, dispersants, wetting agents, preservatives, viscous agents, pH adjusters, coloring agents, flavoring agents, surfactants, and solubilizing agents. When the composition is in liquid form, thickeners such as pectin, xanthan gum, and guar gum can be added. The composition can also be made into coated tablets using a coating agent or into a paste-like glue. Furthermore, even when the composition is prepared into other forms, conventional methods should be followed.

The method of treatment or prevention of the present invention can be implemented based on the above description. Examples of the subject to be administered the compound of formula (I) or formula (IX), or a pharmaceutically acceptable salt thereof, include mammals such as humans.

(Production method)

Hereinafter, the method for producing the compound of the present invention will be described using examples, but the present invention is not limited thereto.

Intermediates and title compounds in the following production methods can also be converted to other compounds included in the present invention by converting their functional groups as appropriate. As a protective group, common protective groups described in the literature (T.W. Greene and P.G.M. Wuts, "Protective Groups in Organic Synthesis", 3rd Ed. John Wiley and Sons, inc., New York (1999)) and the like can be used, and the introduction and removal of protective groups can be performed by methods commonly used in organic synthetic chemistry (e.g., methods described in the above literature) or by methods based thereon. Specifically, examples of protective groups for amino include benzyloxycarbonyl, tert-butoxycarbonyl, acetyl, and benzyl, and examples of protective groups for hydroxy include trialkylsilyl, acetyl, and benzyl.

Starting materials and intermediates for each of the following production methods can be obtained by purchasing commercially available products, synthesizing according to known literature, or synthesizing from known compounds by known methods. Salts of the starting materials and intermediates may also be used as necessary.

The inert solvent in the following production methods means a solvent that does not react with the raw materials, reagents, bases, acids, catalysts, ligands, and the like used in the reaction.

A prodrug of the compound of formula (I) or formula (IX) can be produced by introducing a specific group at the stage from raw material to intermediate, similarly to the above protective group, or by further reaction using the obtained compound of formula (I). The reaction can be carried out by applying a method known to those skilled in the art such as the commonly used esterification, amidation, and dehydration.

(First Production Method)

As a typical production method, the case of a compound represented by general formula (I) in which Het is pyrazole will be described.

[Formula 9]

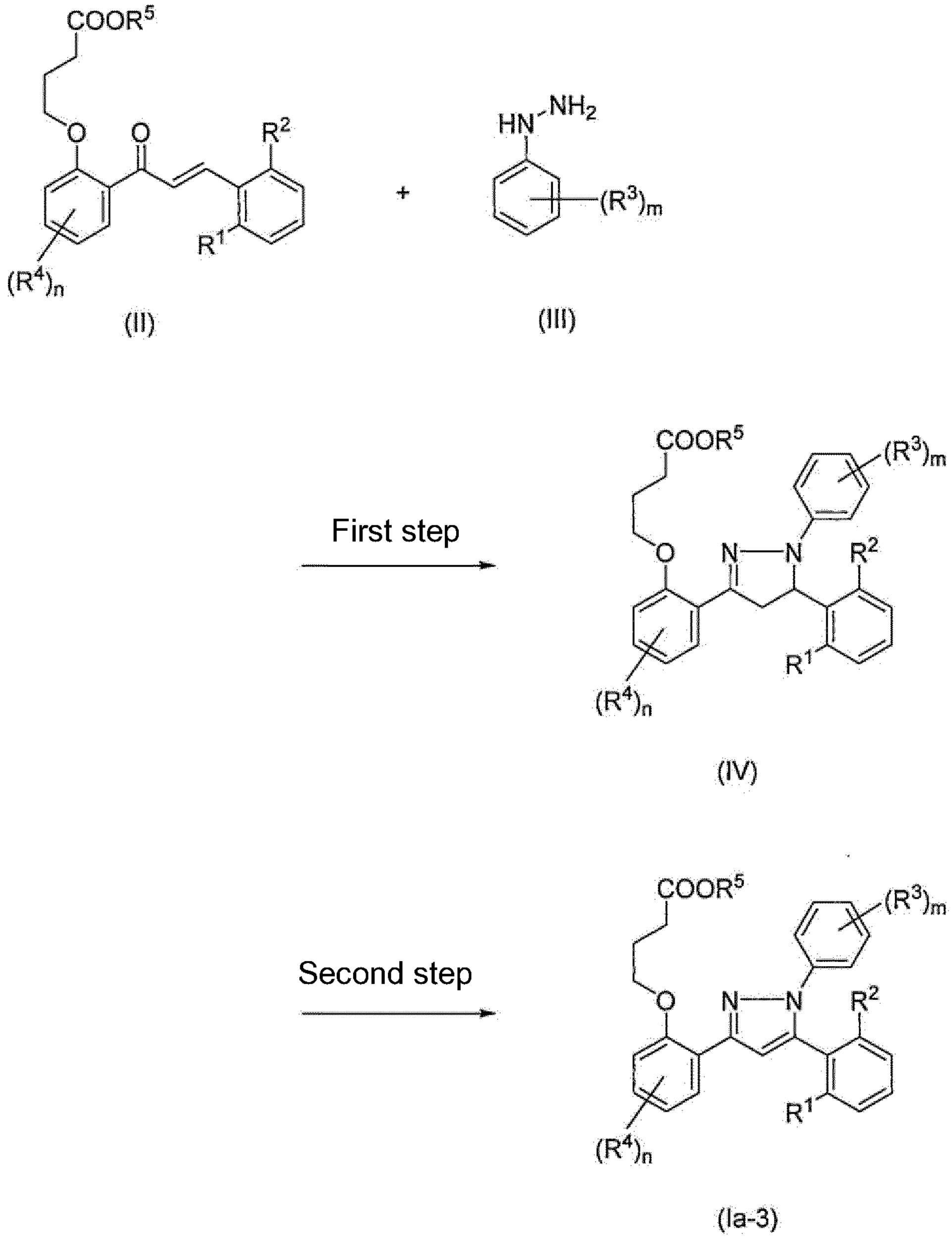


(II) (III) (IV) (Ia-3)

(wherein $R^1$ to $R^5$, m, and n are as already defined)

(First Step)

The present step is a step to obtain a compound of formula (IV) by a cyclization reaction of a compound of formula (II) with a compound of formula (III). In this reaction, the compound of formula (II) and the compound of formula (III) are used in equal amounts or in excess of either, and the mixture thereof is stirred in a reaction-inert solvent or under no solvent, from cooling to heat reflux, for example at room temperature to 120°C, usually for 1 hour to 3 days. Examples of the solvents used here include, but are not limited to, ethers such as diethyl ether, THF, 1,4-dioxane, and 1,2-dimethoxyethane, alcohols such as methanol, ethanol, 1-propanol, 2-propanol, and 1-butanol, water, acetic acid, pyridine, acetonitrile, NMP, DMF, DMSO, and mixtures thereof. It may be advantageous to carry out the reaction in the presence of an organic base such as triethylamine and N,N-diisopropylethylamine, or an inorganic base such as potassium carbonate, sodium carbonate, or cesium carbonate to facilitate the reaction.

(Second Step)

The present step is a step to obtain a compound of formula (Ia-3) by the oxidation of formula (IV). In this reaction,

the compound of formula (IV) and an oxidizing agent are used in equal amounts or in excess of either, and the mixture thereof is stirred in a reaction-inert solvent or under no solvent, from cooling to heat reflux, for example at room temperature to 120°C, usually for 1 hour to 3 days. Examples of the solvents used here include, but are not limited to, ethers such as diethyl ether, THF, 1,4-dioxane, and 1,2-dimethoxyethane, alcohols such as methanol, ethanol, 1-propanol, 2-propanol, and 1-butanol, water, pyridine, acetonitrile, NMP, DMF, DMSO, and mixtures thereof. It may be advantageous to carry out the reaction in the presence of an organic base such as triethylamine and N,N-diisopropylethylamine, or an inorganic base such as potassium carbonate, sodium carbonate, or cesium carbonate to facilitate the reaction. Examples of the oxidizing agent used here include, but are not limited to, oxygen, manganese dioxide, potassium permanganate, sodium tungstate, hydrogen peroxide, and 2,3-dichloro-5,6-dicyano-p-benzoquinone. The addition of a metal such as copper chloride, palladium acetate, and platinum may be advantageous to facilitate the reaction.

(Second Production Method)

The case of a compound represented by general formula (I) in which Het is triazole will be described.

[Formula 10]

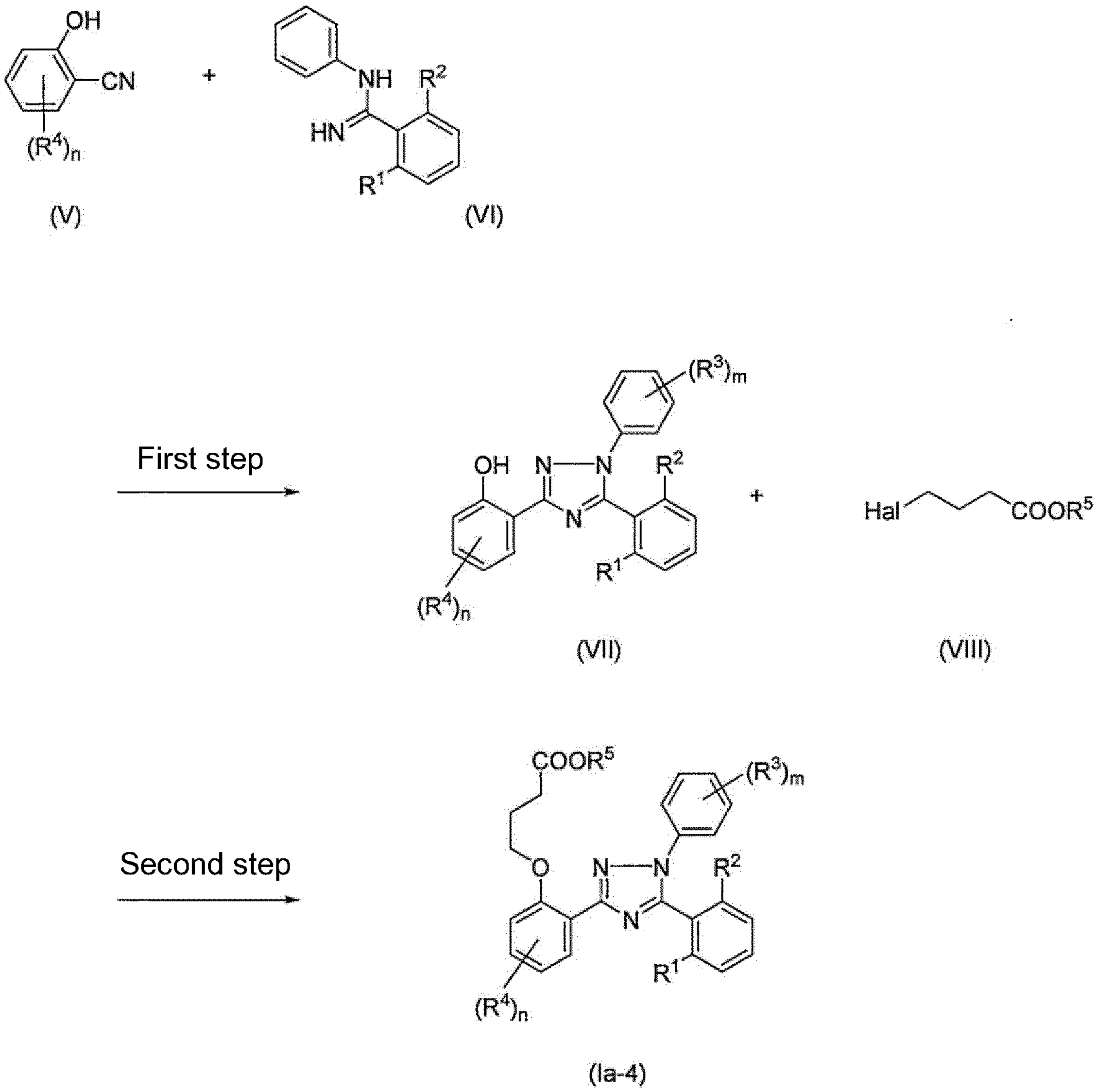


(V) (VI) (VII) (VIII) (Ia-4)

(wherein $R^1$ to $R^5$, m, and n are as already defined, and Hal represents a halogen atom)

(First Step)

The present step is a step to obtain a compound of formula (VII) by a ring-closing reaction of a compound of formula (V) with a compound of formula (VI). In this reaction, the compound of formula (V) and the compound of formula (VI) are used in equal amounts or in excess of either, and the mixture thereof is stirred in a reaction-inert solvent or under no solvent, from cooling to heat reflux, for example at room temperature to 120°C, usually for 1 hour to 3 days. Examples of the solvents used here include, but are not limited to, ethers such as diethyl ether, THF, 1,4-dioxane, and 1,2-dimethoxyethane, alcohols such as methanol, ethanol, 1-propanol, 2-propanol, and 1-butanol, water, acetic acid, pyridine, acetonitrile, NMP, DMF, DMSO, and mixtures thereof. It may be advantageous to carry out the reaction in the presence of an organic base such as triethylamine and N,N-diisopropylethylamine, or an inorganic base such as potassium carbonate, sodium carbonate, or cesium carbonate to facilitate the reaction.

(Second Step)

The present step is a step to obtain a compound of formula (Ia-4) by an etherification reaction of a compound of formula (VII) with a compound of formula (VIII). In this reaction, the compound of formula (VII) and the compound of formula (VIII) are used in equal amounts or in excess of either, and the mixture thereof is stirred in a reaction-inert solvent or under no solvent, from cooling to heat reflux, for example at room temperature to 120°C, usually for 1 hour to 3 days. Examples of the solvents used here include, but are not limited to, ethers such as diethyl ether, THF, 1,4-dioxane, and 1,2-dimethoxyethane, alcohols such as methanol, ethanol, 1-propanol, 2-propanol, and 1-butanol, water, pyridine, acetonitrile, NMP, DMF, DMSO, and mixtures thereof. It may be advantageous to carry out the reaction in the presence of an organic base such as triethylamine and N,N-diisopropylethylamine, or an inorganic base such as potassium carbonate, sodium carbonate, or cesium carbonate to facilitate the reaction.

EXAMPLES

Hereinafter, the present invention will be described in further detail by showing Examples, but the present invention is not limited to these Examples.

The symbols used in the specification of the present application are as follows. Et: ethyl, iPr: isopropyl, DMF: dimethylformamide, DMSO: dimethyl sulfoxide, THF: tetrahydrofuran, NMP: N-methyl-2-pyrrolidone.

Example 1: 4-(4-fluoro-2-(1-phenyl-5-(2-(trifluoromethyl)phenyl)-1H-pyrazol-3-yl)phenoxy)butanoic acid

[First Step] Preparation of (E)-1-(5-fluoro-2-hydroxyphenyl)-3-(2-(trifluoromethyl)phenyl)prop-2-en-1-one

[Formula 11]

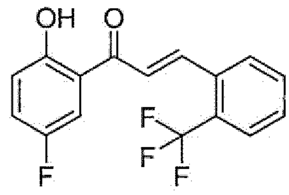

Potassium hydroxide (37 g) was added to ethanol (700 mL) and dissolved. To this solution, 2-acetyl-4-fluorophenol (20 g) was added at room temperature, followed by further addition of 2-(trifluoromethyl)benzaldehyde (25 g). The resulting mixture was stirred at room temperature for 3 hours, then cooled with ice, and 1M hydrochloric acid solution (440 mL) was added dropwise. The precipitated solid was collected by filtration and washed with water. The resulting solid was dried under reduced pressure at 40°C to give the title orange compound (33 g, 81% yield).

$^1$H NMR (400 MHz, $CDCl_3$) δ (ppm): 12.37 (1H, s), 8.29 (1H, d, J = 15.6 Hz), 7.86 (1H, d, J = 8.0 Hz), 7.76 (1H, d, J = 7.6 Hz), 7.65 (1H, t, J = 7.6 Hz), 7.54-7.57 (2H, m), 7.48 (1H, d, J = 15.6 Hz), 7.24-7.29 (1H, m), 7.02 (1H, dd, J = 4.8, 9.6 Hz).

Mass spectrometry ESI(-): 309 [m-H] (calculated value: 310).

[Second Step] Preparation of ethyl (E)-4-(4-fluoro-(2-(3-(2-trifluoromethyl)phenyl)prop-2-enoyl)phenoxy)butanoate

[Formula 12]

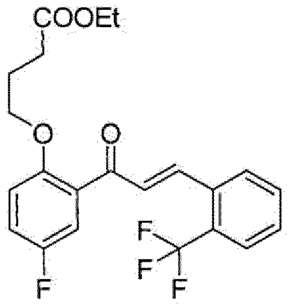

Potassium carbonate (13 g) was added to a solution of (E)-1-(5-fluoro-2-hydroxyphenyl)-3-(2-(trifluoromethyl)phenyl)prop-2-en-1-one (23 g) in DMF (120 mL), and the mixture was stirred at room temperature. Ethyl 4-bromobutanoate (12 mL) was added to the resulting mixture and stirred at room temperature for 3 hours. Water (230 mL) was added to the reaction mixture, followed by extraction with ethyl acetate. Furthermore, the aqueous layer was extracted twice with ethyl acetate, and the organic layers were combined and washed with water. The organic layer was dehydrated and concentrated under reduced pressure to give the title compound (33 g). This was used in the next step without purification.
$^1$H NMR (400 MHz, $CDCl_3$) δ (ppm): 7.97 (1H, d, J = 15.7 Hz), 7.8 (1H, d, J = 7.6 Hz), 7.71 (1H, d, J = 7.6 Hz), 7.59 (1H, t, J = 7.6 Hz), 7.49 (1H, t, J = 7.6 Hz), 7.34-7.38 (2H, m), 7.16 (1H, m), 6.93 (1H, dd, J = 9.2 and 4.4 Hz), 4.03-4.08 (4H, m), 2.40 (2H, t, J = 7.3), 2.04-2.11 (2H, m), 1.18 (3H, t, J = 7.1 Hz).
Mass spectrometry ESI(+): 425 [m+H], 447 [m+Na], 463 [m+K], 871 [2m+Na] (calculated value: 424).

[Third Step] Preparation of ethyl 4-(4-fluoro-2-(1-phenyl-5-(2-(trifluoromethyl)phenyl)-4,5-dihydro-1H-pyrazol-3-yl)phenoxy)butanoate

[Formula 13]

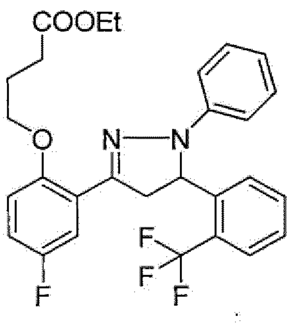

Phenylhydrazine (11 mL) was added to a solution of ethyl (E)-4-(4-fluoro-(2-(3-(2-trifluoromethyl)phenyl)prop-2-enoyl)phenoxy)butanoate (33 g) in ethanol (310 mL), and the mixture was stirred under heat reflux for 2 hours. The reaction mixture was cooled with ice and the precipitated solid was collected by filtration. The resulting solid was washed with cooled ethanol to give the title yellowish white compound (18 g, 47% yield).
$^1$H NMR (400 MHz, $CDCl_3$) δ (ppm): 7.71-7.75 (2H, m), 7.33-7.44 (3H, m), 7.15-7.19 (2H, m), 6.70-6.92 (3H, m), 6.76-6.79 (2H, m), 5.67 (1H, q, J = 6.3 Hz), 3.92-4.14 (5H, m), 3.23 (1H, dd, J = 18.1, 5.7 Hz), 2.41 (2H, t, J = 7.3 Hz), 2.05 (2H, q, J = 6.7 Hz), 1.23 (3H, t, J = 7.1 Hz).
Mass spectrometry ESI(+): 515, 537, 553 (calculated value: 514).

[Fourth Step] Preparation of ethyl 4-(4-fluoro-2-(1-phenyl-5-(2-(trifluoromethyl)phenyl)-1H-pyrazol-3-yl)phenoxy)butanoate

[Formula 14]

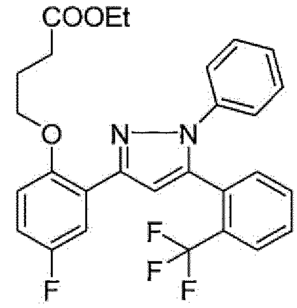

Manganese dioxide (5 g) was added to a solution of ethyl 4-(4-fluoro-2-(1-phenyl-5-(2-(trifluoromethyl)phenyl)-4,5-dihydro-1H-pyrazol-3-yl)phenoxy)butanoate (12 g) in ethyl acetate (240 mL), and the mixture was stirred under heat reflux for 17 hours. The insoluble material in the reaction mixture was removed by filtration and the filtrate was concentrated to give the title compound (12 g) as a colorless to yellow oil. This was used in the next step without purification.

$^1$H NMR (400 MHz, $CDCl_3$) $\delta$ (ppm): 7.91 (1H, dd, J = 10.1, 3.2 Hz), 7.77 (1H, d, J = 7.3 Hz), 7.39-7.48 (2H, m), 7.18-7.31 (6H, m), 7.14 (1H, s), 6.93-6.98 (1H, m), 6.87-6.90 (1H, m), 4.05-4.12 (4H, m), 2.52 (2H, t, J = 7.3 Hz), 2.14-2.17 (2H, m), 1.19 (3H, t, J = 7.1 Hz).

Mass spectrometry ESI(+): 513, 535, 551 (calculated value: 512).

[Fifth Step] Preparation of 4-(4-fluoro-2-(1-phenyl-5-(2-(trifluoromethyl)phenyl)-1H-pyrazol-3-yl)phenoxy)butanoic acid

[Formula 15]

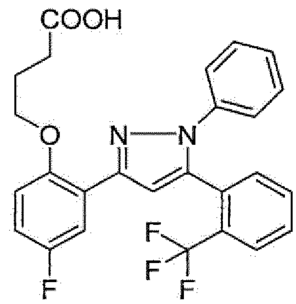

Ethanol (60 mL) and 1M sodium hydroxide solution (60 mL) were added to ethyl 4-(4-fluoro-2-(1-phenyl-5-(2-(trifluoromethyl)phenyl)-1H-pyrazol-3-yl)phenoxy)butanoate (12 g), and the mixture was stirred overnight at room temperature. 1M hydrochloric acid solution (5 mL) was added dropwise and the precipitated solid was collected by filtration. The solid was washed with cooled ethanol and dried. The title white compound (10 g, 91% yield) was obtained.

$^1$H NMR (400 MHz, $CDCl_3$) $\delta$ (ppm): 7.86 (1H, dd, J = 9.6, 3.2 Hz), 7.75-7.77 (1H, m), 7.40-7.48 ( (2H, m), 7.17-7.30 (6H, m), 7.09 (1H, s), 6.95-7.00 (1H, m), 6.87-6.91 (1H, m), 4.08 (2H, t, J = 6.0 Hz), 2.57 (2H, t, J = 7.2 Hz), 2.11-2.18 (2H, m).

ESI(+): 485 [m+H], 969 [2m+H], 991 [2m+Na] (calculated value: 484).

Example 2: 4-(2-(1-phenyl-5-(2-(trifluoromethyl)phenyl)-1H-pyrazol-3-yl)phenoxy)butanoic acid

[First Step] Preparation of (E)-1-(2-hydroxyphenyl)-3-(2-(trifluoromethyl)phenyl)prop-2-en-1-one

[Formula 16]

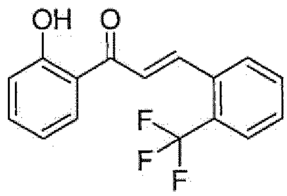

Potassium hydroxide (4.12 g) was added to ethanol (70 mL) and dissolved. To this solution, 2-hydroxyacetophenone (2 g) was added at room temperature, followed by further addition of 2-(trifluoromethyl)benzaldehyde (2.81 g). The resulting mixture was stirred at room temperature for 17 hours, then cooled with ice, and 2M hydrochloric acid solution was added dropwise. The precipitated solid was collected by filtration and washed with water, then dried under reduced pressure at 40°C to give the orange (E)-1-(2-hydroxyphenyl)-3-(2-(trifluoromethyl)phenyl)prop-2-en-1-one (3.2 g (75% yield)).

$^1$H NMR (400 MHz, $CDCl_3$) δ (ppm): 12.65 (1H, s), 8.23-8.28 (1H, m), 7.83-7.90 (2H, m), 7.74 (1H, d, J = 7.8 Hz), 7.03 (1H, d, J = 8.7 Hz, 6.92-6.96 (1H, m).

Mass spectrometry ESI(-): 291, ESI(+): 293 (calculated value: 292).

[Second Step] Preparation of ethyl (E)-4-(2-(3-(2-trifluoromethyl)phenyl)prop-2-enoyl)phenoxy)butanoate

[Formula 17]

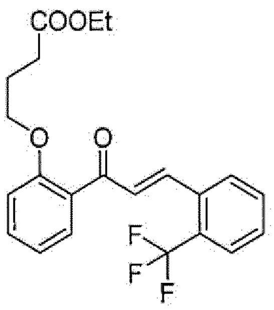

Potassium carbonate (0.3 g) was added to a solution of (E)-1-(2-hydroxyphenyl)-3-(2-(trifluoromethyl)phenyl) prop-2-en-1-one (0.5 g) in DMF (2.5 mL), and the mixture was stirred at room temperature. Ethyl 4-bromobutanoate (0.4 mL) was added to the resulting mixture and stirred at room temperature for 17 hours. Water (5 mL) was added, followed by extraction with ethyl acetate. Furthermore, the aqueous layer was extracted twice with ethyl acetate, and the organic layers were combined and washed with water. The organic layer was dehydrated and concentrated under reduced pressure to give a white solid (0.7 g) as the title compound. This was used in the next step without purification.

$^1$H NMR (400 MHz, $CDCl_3$) δ (ppm): 7.96 (1H, dd, J = 1.8, 15.6 Hz), 7.80 (1H, d, J = 7.8 Hz), 7.70 (1H, d, J = 7.8 Hz), 7.64 (1H, dd, J = 1.8, 7.8 Hz), 5.58 (1H, t, J = 7.5 Hz), 7.44-7.49 (2H, m), 7.37 (1H, d, J = 15.6 Hz), 7.04 (1H, t, J = 7.5 Hz), 6.97 (1H, d, J = 8.2 Hz), 4.02-4.11 (4H, m), 2.41 (2H, t, J = 7.3 Hz), 2.05-2.12 (2H, m), 1.18 (3H, t, J = 7.1 Hz).

Mass spectrometry ESI(+): 407 [m+H], 429 [m+Na], 445 [m+K], 835 [2m+Na] (calculated value: 406).

[Third Step] Preparation of ethyl 4-(2-(1-phenyl-5-(2-(trifluoromethyl)phenyl)-4,5-dihydro-1H-pyrazol-3-yl)phenoxy)butanoate

[Formula 18]

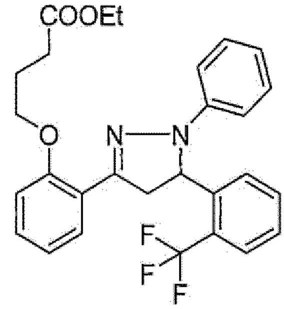

Phenylhydrazine (3.2 mL) was added to a solution of ethyl (E)-4-(2-(3-(2-trifluoromethyl)phenyl)prop-2-enoyl) phenoxy)butanoate (13 g) in ethanol (70 mL), and the mixture was stirred under heat reflux for 17 hours. The reaction mixture was cooled with ice and the precipitated solid was collected by filtration. The solid was washed with cooled ethanol to give the title yellowish white compound (3 g, 19% yield).

$^1$H NMR (400 MHz, $CDCl_3$) δ (ppm): 7.98 (1H, dd, J = 1.8, 7.8 Hz), 7.71 (1H, d, J = 7.8 Hz), 7.23-7.44 (4H, m), 7.14-7.19 (2H, m), 6.97-7.00 (3H, m), 6.86 (1H, d, J = 8.2 Hz), 6.76 (1H, t, J = 7.3 Hz), 5.65 (1H, m), 3.97-4.14 (5H, m), 3.24 (1H, dd, J = 5.7, 17.6 Hz), 2.41 (2H, t, J = 7.3 Hz), 2.04-2.07 (2H, m), 1.23 (3H, t, J = 7.1 Hz).

Mass spectrometry ESI(+): 497, 519, 535 (calculated value: 496).

[Fourth Step] Preparation of ethyl 4-(2-(1-phenyl-5-(2-(trifluoromethyl)phenyl)-1H-pyrazol-3-yl)phenoxy)butanoate

[Formula 19]

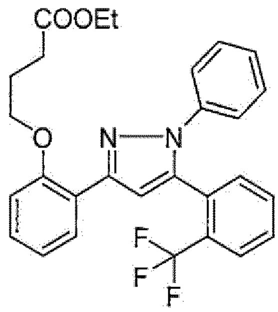

Manganese dioxide (5 g) was added to a solution of ethyl 4-(2-(1-phenyl-5-(2-(trifluoromethyl)phenyl)-4,5-dihydro-1H-pyrazol-3-yl)phenoxy)butanoate (2.9 g) in ethyl acetate (30 mL), and the resulting mixture was stirred under heat reflux for 17 hours. The insoluble material was removed from the reaction mixture by filtration and the filtrate was concentrated to give the title compound (2.9 g) as a solid. This was used in the next step without purification.

$^1$H NMR (400 MHz, $CDCl_3$) δ (ppm): 8.14 (1H, dd, J = 1.6, 7.5 Hz), 7.74 (1H, d, J = 7.3 Hz), 7.34-7.43 (2H, m), 7.13-7.32 (7H, m), 7.10 (1H, s), 7.01 (1H, t, J = 7.4 Hz), 6.94 (1H, d, J = 8.4 Hz), 4.04-4.09 (4H, m), 2.51 (2H, t, J = 7.3 Hz), 2.11-2.18 (2H, m), 1.17 (3H, t, J = 7.2 Hz).

Mass spectrometry ESI(+): 495, 517, 533 (calculated value: 494).

[Fifth Step] Preparation of 4-(2-(1-phenyl-5-(2-(trifluoromethyl)phenyl)-1H-pyrazol-3-yl)phenoxy)butanoic acid

[Formula 20]

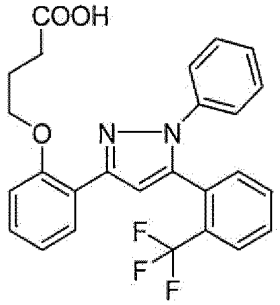

Ethanol (60 mL) and 1M sodium hydroxide solution (60 mL) were added to ethyl 4-(2-(1-phenyl-5-(2-(trifluoromethyl)phenyl)-1H-pyrazol-3-yl)phenoxy)butanoate (2.9 g), and the resulting mixture was stirred overnight at room temperature. To the reaction mixture, 5 mL of 1M hydrochloric acid solution was added dropwise. The precipitated solid was collected by filtration, washed with cooled ethanol and dried. The title compound (2.5 g, 90% yield) was obtained as a white solid.

$^1H$ NMR (400 MHz, $CDCl_3$) δ (ppm): 8.08 (1H, dd, J = 1.6, 7.5 Hz), 7.75-7.77 (1H, m), 7.40-7.45 (2H, m), 7.19-7.33 (8H, m), 7.03-7.07 (2H, m), 6.96-6.99 (1H, m), 4.13 (2H, t, J = 5.9 Hz), 2.58 (2H, t, J = 7.1 Hz), 2.15-2.20 (2H, m).

Mass spectrometry ESI(+): 467, 489, 505, 955, 971 (calculated value: 466).

Example 3: 4-(2-(1-phenyl-5-(2-(trifluoromethyl)phenyl)-1H-1,2,4-triazol-3-yl)phenoxy)butanoic acid

[First Step] Preparation of N-phenyl-2-(trifluoromethyl)benzenecarboximidamide

[Formula 21]

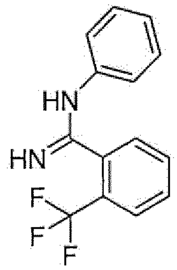

55% sodium hydride (in oil, 1.5 g) was added to a solution of aniline (2.7 g) in DMSO (25 mL), and the mixture was stirred at room temperature for 5 minutes. 2-Trifluoromethyl cyanobenzene (10 g) was added to the resulting mixture and stirred overnight. Water was added to the reaction mixture, followed by extraction with ethyl acetate. The organic layer was washed with water, and concentrated under reduced pressure. The concentrate was purified by silica gel column chromatography (chloroform/methanol = 100:1) to give the title compound (3.5 g, 45% yield).

$^1H$ NMR (400 MHz, $CDCl_3$) δ (ppm): 7.13 (d, J = 7.7Hz, 1H), 7.70-7.47 (m, 3H), 7.42-7.26 (m, 2H), 7.12-6.92 (m, 3H).

Mass spectrometry ESI(+): 265 (calculated value: 264).

[Second Step] Preparation of 2-(1-phenyl-5-(2-(trifluoromethyl)phenyl)-1H-1,2,4-triazol-3-yl)phenol

[Formula 22]

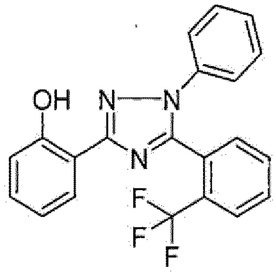

A mixture of 2-cyanophenol (4.3 g), N-phenyl-2-(trifluoromethyl)benzenecarboximidamide (2.4 g), 1,10-phenanthroline (41 mg), copper acetate (41 mg), and sodium carbonate (3.9 g) in toluene (100 mL) was stirred under heat reflux for 17 hours. After cooling, water was added to the reaction mixture, followed by two extractions with ethyl acetate. The organic layers were combined and washed with water, and the solvent was removed under reduced pressure to give a yellow oil. Purification by column chromatography (chloroform/methanol = 100:1) afforded 2-(1-phenyl-5-(2-(trifluoromethyl)phenyl)-1H-1,2,4-triazol-3-yl)phenol (120 mg).

$^1$H NMR (400 MHz, $CDCl_3$) $\delta$ (ppm): 9.68 (1H, s), 7.81-7.33 (11H, m), 7.03-7.07 (2H, m), 6.96-6.99 (1H, m).

Mass spectrometry ESI(+): 382, 404 (calculated value: 381).

[Third Step] Preparation of 4-(2-(1-phenyl-5-(2-(trifluoromethyl)phenyl)-1H-1,2,4-triazol-3-yl)phenoxy)butanoic acid

[Formula 23]

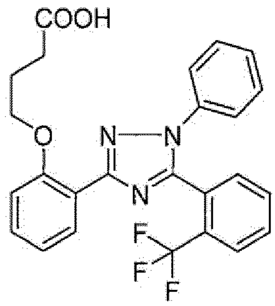

A mixture of 2-(1-phenyl-5-(2-(trifluoromethyl)phenyl)-1H-1,2,4-triazol-3-yl)phenol (100 mg), potassium carbonate (120 mg), and ethyl 4-bromobutanoate (200 $\mu$L) in DMF (5 mL) was stirred at 90°C for 2 hours. The reaction mixture was extracted twice with ethyl acetate. The organic layers were combined, washed with water, and dehydrated, and then the solvent was removed.

Ethanol (5 mL) and 2M sodium hydroxide solution (5 mL) were added to the resulting product, and the mixture was stirred at room temperature for 24 hours. The reaction mixture was neutralized with 2M hydrochloric acid solution and extracted twice with ethyl acetate. The organic layers were combined, washed with water, and dehydrated, and then the solvent was removed. The concentrate was dissolved in ethanol and decolorized with activated carbon. Water was added dropwise and the precipitated solid was collected by filtration, washed and dried to give the title compound (40 mg).

$^1$H NMR (400 MHz, $CDCl_3$) $\delta$ (ppm): 7.81-7.33 (12H, m), 7.05-7.08 (2H, m), 4.23 (2H, m), 2.60 (2H, m), 2.25-2.30 (2H, m).

Mass spectrometry ESI(+): 468, 490, 506 (calculated value: 467).

Example 4: 4-(4-fluoro-2-(1-(4-isopropylphenyl)-5-(2-(trifluoromethyl)phenyl)-1H-pyrazol-3-yl)phenoxy)butanoic acid

[Formula 24]

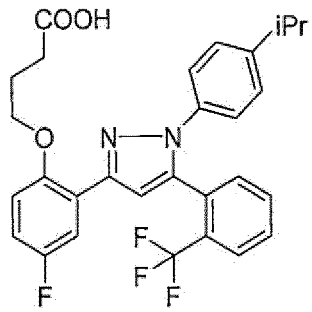

The title compound was prepared as a yellowish white solid by the same method as in Example 1, except that 4-isopropylphenylhydrazine was used instead of phenylhydrazine in step 3 of Example 1.

$^1H$ NMR (400 MHz, $CDCl_3$) δ (ppm): 7.84 (1H, dd, J = 9.6, 3.2 Hz), 7.75-7.77 (1H, m), 7.40-7.48 (2H, m), 7.17-7.25 (3H, m), 7.09-7.12 (2H, m), 6.94-6.99 (1H, m), 6.87-6.90 (1H, m), 4.08 (2H, t, J = 5.9 Hz), 2.81-2.88 (1H, m), 2.57 (2H, J = 7.3 Hz), 2.11-2.18 (2H, m), 1.20 (6H, d, J = 7.3 Hz).

Mass spectrometry ESI(+): 527, 549, 565 (calculated value: 526).

Example 5: 4-(2-(1-(4-isopropylphenyl)-5-(2-(trifluoromethyl)phenyl)-1H-pyrazol-3-yl)phenoxy)butanoic acid

[Formula 25]

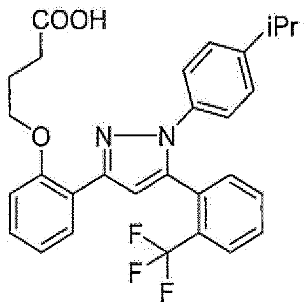

The title compound was prepared as a white solid by the same method as in Example 1, except that 4-isopropylphenylhydrazine was used instead of phenylhydrazine in step 3 of Example 2.

$^1H$ NMR (400 MHz, $CDCl_3$) δ (ppm): 7.84 (1H, dd, J = 9.6, 3.5 Hz), 7.75-7.77 (1H, m), 7.40-7.38 (3H, m), 7.17-7.25 (3H, m), 7.09-7.12 (2H, m), 6.94-6.95 (1H, m), 6.86-6.91 (1H, m), 4.11 (2H, t, J = 5.7 Hz), 2.81-2.89 (1H, m), 2.59 (2H, J = 7.3 Hz), 2.10-2.17 (2H, m), 1.20 (6H, d, J = 7.3 Hz).

Mass spectrometry ESI(+): 509, 531, 547 (calculated value: 508).

Test Example 1: Evaluation of FABP ligand affinity using ANS

GST-FABP3, GST-FABP4, GST-FABP5, and GST-FABP7 were each expressed in E. coli [BL21 (DE3) strain], and then affinity purification was performed using a glutathione column.

Cloning of GST-FABP3, GST-FABP4, GST-FABP5 and GST-FABP7 vectors was performed by the following method. After reverse transcription of mRNA isolated from mouse heart into cDNA, the FABP3 gene (RefSeqID: NM_010174.1) and FABP4 gene (RefSeqID: NM_024406.2) were each amplified by PCR. These cDNAs were each inserted between the BamHI/EcoRI cleavage sites of a pGEX-2T vector (GE HealthCare Japan, Tokyo) using a DNA ligation kit (Takara Bio, Kusatsu) and an In-fusion kit (Takara Bio USA, CA, USA) to prepare vectors.

The nucleotide sequences of GST-FABP3, GST-FABP4, GST-FABP5, and GST-FABP7 that were sequenced are listed in Fig. 1 (the first and last GGATTC and GAATTC are restriction enzyme cleavage sites).

Purification of GST-binding proteins was performed using a GST purification kit (Takara Bio USA). After the E. coli were collected by centrifugation, the provided extraction buffer and aluminum oxide powder were added, and the mixture was crushed in a mortar. The centrifuged supernatant was added to the provided glutathione column and allowed to stand

on ice for 30 minutes. The supernatant in the column was then discarded, and washing was performed with extraction buffer, followed by elution with elution buffer containing glutathione. The protein concentration of the elution product was calculated from the absorbance at 280 nm and then used for the ANS assay.

1-anilinonaphthalene-8-sulfonic acid (ANS, final concentration: 4 mM, in a solution of 10 mM $KH_2PO_4$ and 40 mM KCl at pH 7.4), which binds to FABP proteins and emits fluorescence, and FABP proteins (final concentration: 0.4 mM), as well as various ligands at concentrations of 0 nM, 100 nM, 1,000 nM, 2,000 nM, and 4,000 nM (final) were incubated for 2 min, and then the fluorescence of ANS was measured (Ex/Em = 355 nm/460 nm). The fluorescence intensity at each ligand concentration was converted to a relative value (%) to the ANS fluorescence intensity in the absence of ligand, and then the dissociation constant Kd (nM) was calculated by non-regression analysis using the following equation.

$$F = F_0 - \{[1 + (P_t + L_t) Ka - [(P_t - L_t)^2 Ka^2 + 2(P_t+L_t) Ka + 1]^{1/2}]/[2P_tKa]\} (F_0 - F_{max})$$

F: Relative fluorescence intensity (%) under certain conditions;
$F_0$: Fluorescence intensity in the absence of ligand (= 100);
$P_t$: FABP protein concentration (= 400 nM);
$L_t$: Ligand concentration (= 100, 1,000, 2,000, 4,000 nM);
Ka: inverse of the dissociation constant Kd ($nM^{-1}$);
$F_{max}$*: relative fluorescence intensity when FABP is fully occupied by ligands.

The compounds of Examples 1 to 5 (referred to as Example Compound 1 to Example Compound 5, respectively) and the known compound LIGAND 1 (described in WO2017/171053) were used as ligands.

The dissociation constants Kd calculated based on the measurement results are shown in Table 1. Example Compounds 1 to 5 were found to have smaller dissociation constants Kd than LIGAND 1, particularly for FABP3.

[Table 1-1]

| Table 1. Kd value of FABP for each ligand (nM) | | | | | |
|---|---|---|---|---|---|
| Example Compound | Structure | GST-FABP3 | GST-FABP4 | GST-FABP5 | GST-FABP7 |
| 1 | 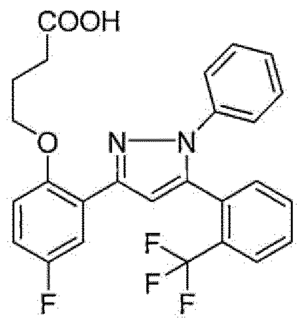 | 24.0±7.2 | 238.2 ± 45.7 | 410.3 ± 70.0 | No binding |
| 2 | 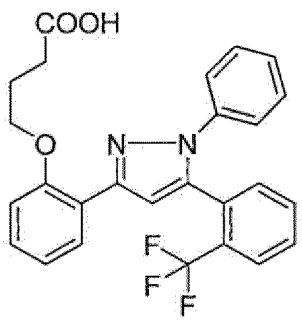 | 11.7±8.8 | 103.9 ± 36.3 | 189.0 ± 24.5 | 2048.3 ± 35.2 |

(continued)

| Table 1. Kd value of FABP for each ligand (nM) | | | | | |
|---|---|---|---|---|---|
| Example Compound | Structure | GST-FABP3 | GST-FABP4 | GST-FABP5 | GST-FABP7 |
| 3 | 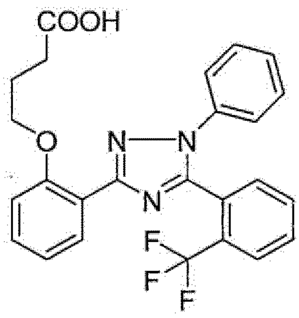 | 159.9 ± 34.1 | 368.6 ± 44.1 | Not measured | No binding |
| 4 | 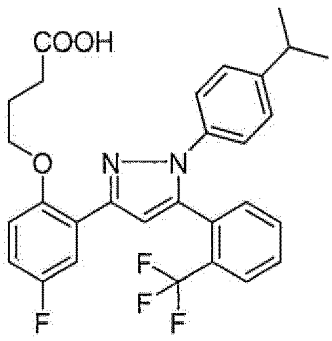 | 113.4 ± 3.1 | 2182.3 ± 457.7 | 1352.7 ± 204.1 | No binding |

[Table 1-2]

| | | | | | |
|---|---|---|---|---|---|
| 5 | 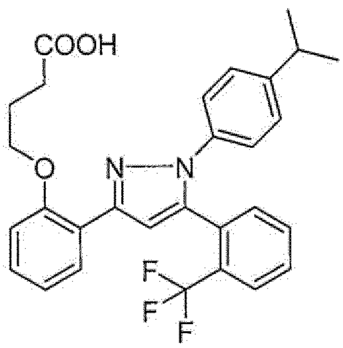 | 54.8±4.5 | 434.0 ± 10.9 | 1824.0 ± 391.7 | No binding |
| LIGAND 1 | 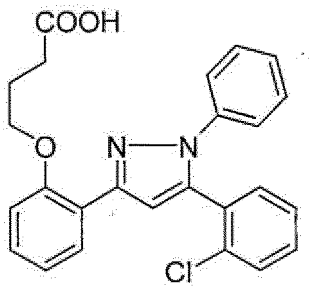 | 302.8 ± 130.3 | 228.9 ± 39.9 | 324.1 ± 45.2 | Not measured |

The Kd values in the above table are indicated as the mean ± SE of three measurements. If the Kd value was 1,000,000 or more, it was indicated as "no binding". Example Compounds 1 to 5 in the table may also be referred to as Compounds 1 to 5, respectively.

Test Example 2: Cognitive function evaluation test using PD model animals

The dopaminergic neurotoxin 1-methyl-4-phenyl-1,2,3,6-tetrahydropyridine (MPTP, 25 mg/kg, i.p., purchased from Sigma-Aldrich (St Louis, MO)) was administered once daily for five consecutive days (Days 1 to 5) to 8- to 10-week-old male C57BL6 N mice, and on the twentieth day after the start of the test (Day 20), the mice exhibited PD-like symptoms. The model animals were given a solvent or FABP3 ligand (compound of Example 1 (Example Compound 1), 0.05, 0.1 and 0.3 mg/kg, p.o., n = 7 per group) once daily for two weeks starting from 20 days after the start of the test (Day 20). Cognitive function was evaluated by a passive avoidance task 34 days after the start of the test (Day 34). In the passive avoidance task, the mice were placed in a bright room during the training trial, and electrical stimulation (0.3 mA, 2 seconds) was given when the mice entered a dark room. After twenty-four hours, the mice were placed again in the bright room, and the time until they entered the dark room (corresponding to the vertical axis Latency (s) in Fig. 2) was measured. The results are shown in Fig. 2. A concentration-dependent prolongation of the waiting time was confirmed in the mouse group given ligand. Furthermore, the latency of the vehicle (veh) was confirmed to be significantly different compared to that of the saline, and therefore the method of the present Test Example can be considered appropriate.

In the above model mouse preparation, the normal mouse group that was given saline instead of MPTP is indicated as "saline" in the figure, and the group that was given 0.5% carboxymethyl cellulose instead of FABP3 ligand is indicated as "vehicle (veh)."

Test Example 3: Cognitive function evaluation test using PD model animals

Cognitive function was evaluated by a novel object recognition task 34 days after the start of the test (Day 34) in model mice treated as in Test Example 2, except that the compound of Example 2 (Example Compound 2; 0.03, 0.1, and 0.3 mg/kg) was administered as the ligand for FABP3. In the novel object recognition task, objects of the same shape were placed to be memorized by the mice in the training trial, and in the test trial, one of the objects was replaced with a novel object, and the rate of contact with both objects (corresponding to the vertical axis Discrimination index (%) in Fig. 3) was evaluated. The results are shown in Fig. 3. In the novel object recognition task, there was a large difference between the rates of contact with the novel and the known objects in the ligand-treated group, confirming a trend of improvement in cognitive function.

Test Example 4: Motor function evaluation test using PD model animals

Motor function was evaluated by performing a rotarod task and a beam walking task 34 days after the start of the test (Day 34) in model mice treated as in Test Example 2, except that Example Compound 2 (0.03, 0.1, and 0.3 mg/kg) or LIGAND 1 (compound represented by the following formula; 0.3 mg/kg) was administered as the ligand for FABP3. In the rotarod task, the mice were placed on a roller, and the time until the mice fell when the roller was rotated at a speed of 20 rpm (Latency, corresponding to the vertical axis Latency (s) in Fig. 4) was measured. In the beam walking task, the mice were placed on a thin board and allowed to walk, and the number of foot slips before reaching the goal box (corresponding to the vertical axis Number of foot slips in Fig. 5) was measured.

[Formula 26]

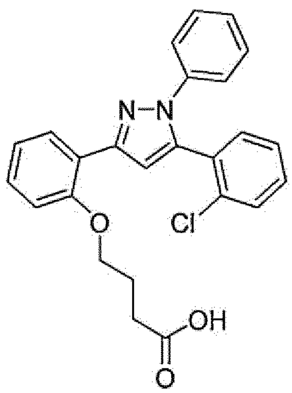

(LIGAND 1)

The results are shown in Figs. 4 and 5. In the rotarod task, improvement in motor function was confirmed in the groups given Example Compound 2, even in the group with the lowest dosage. No improvement was observed in the LIGAND 1-treated group.

In the beam walking task, improvement in motor function was confirmed in all the groups given Example Compound 2. Improvement was also confirmed in the LIGAND 1-treated group.

Test Example 5: Dopaminergic neuroprotection evaluation test using PD model animals

Perfusion fixation was performed 34 days after the start of the test (Day 34) on model mice treated as in Test Example 2, except that Example Compound 2 (0.1, 0.3, and 1.0 mg/kg) or LIGAND 1 (0.3 mg/kg) was administered as the ligand for FABP3, and 50-μm-thick brain sections containing the substantia nigra region (SNpc) and brain sections containing the ventrotegmental area (VTA) were prepared.

These were reacted with TH antibodies (manufactured by Immunostar, mouse monoclonal antibody 22941, 1:1000), α-synuclein antibodies (manufactured by Santa Cruz, rabbit polyclonal antibody SC-70110R, 1:200), and FABP3 antibodies (to be confirmed) and detected with fluorescently labeled secondary antibodies (Alexa 594 anti-mouse IgG (manufactured by Jackson ImmunoResearch, 1:500)). The results for the substantia nigra region are shown in Figs. 6, 7, 10, 11, 14, and 15, and for the ventrotegmental area in Figs. 8, 9, 12, 13, 16, and 17 (the vertical axis Number of $TH^+$ cells in Figs. 6 and 8 corresponds to the number of dopaminergic neurons. The vertical axis Number of double-positive cells/$TH^+$ positive cells (%) in Figs. 11 and 13 corresponds to the percentage of dopaminergic neurons that contain phosphorylated α-synuclein. The vertical axis Triple-positive cells/TH-positive cells in Figs. 15 and 17 corresponds to the percentage of dopaminergic neurons that contain both FABP3 and phosphorylated α-synuclein).

The results shown in Figs. 6 to 9 confirm that Example Compound 2 suppresses the decrease in dopaminergic neurons in MPTP-treated mice. On the other hand, no improvement was observed in the LIGAND 1-treated group. In addition, the results shown in Figs. 10 to 13 confirm that the increased phosphorylation level of α-synuclein in dopaminergic neurons of MPTP-treated mice is reduced by the administration of Example Compound 2. Furthermore, the results shown in Figs. 14 to 17 confirm that the localization of FABP3 and phosphorylated α-synuclein in dopaminergic neurons, caused by MPTP administration to the mice, is reduced by the administration of Example Compound 2. Given that phosphorylation of α-synuclein increases α-synuclein aggregation (Samuel, F. et al., J. Biol. Chem. 291 (2016) 4374-4385) and that α-synuclein aggregates when FABP3 and α-synuclein are both present (Fukui, N. et al., J. Biol. Chem. 296 (2021) 100663), these results show that Example Compound 2 has an improving effect on α-synuclein aggregation, which is useful for improving Parkinson's disease (Fig. 25). In light of a report showing that α-synuclein aggregates propagate (Lee, V. M. Y. et al., J. Exp. Med. Vol. 209, No. 5, 975-986), Example Compound 2 may have a suppressive effect on the propagation of α-synuclein aggregates by improving α-synuclein aggregation. Furthermore, in light of a report that phosphorylated α-synuclein more easily propagates than wild-type α-synuclein (Li, Y-M. et al., Sci Rep. 2016; 6, 37130), Example Compound 2 may have a suppressive effect on α-synuclein propagation by reducing the phosphorylation level of α-synuclein. Thus, it can be said that Example Compound 2 protected dopaminergic neurons from phosphorylated α-synuclein induced by MPTP. Furthermore, it can be said that the interaction between phosphorylated α-synuclein and FABP3 in dopaminergic neurons was inhibited by Example Compound 2.

Test Example 6: Evaluation of cerebral neuroprotection using a focal cerebral ischemia model

Male ICR mice (5 weeks old, 25-30 g) were purchased from Japan SLC, Inc. (Shizuoka, Japan). The animals were reared under constant temperature and humidity conditions, in a 12-hour light/dark cycle (light on: 09:00-21:00), and fed ad libitum. All mice were anesthetized with a combination of 0.3 mg/kg medetomidine, 4.0 mg/kg midazolam, and 5.0 mg/kg butorphanol. A silicone-coated 6-0 suture (Doccol Corporation, USA) was inserted from the right external carotid artery into the internal carotid artery up to the origin of the middle cerebral artery, and left in place for 2 hours, then the suture was removed and reperfusion was performed to prepare a mouse model of focal cerebral ischemia (tMCAO). Mice in the sham surgery group underwent the same treatment except for the insertion of the suture. After reperfusion, the mice were sacrificed at a specified time. During the I/R operation, the core body temperature of the mice was maintained at 37°C using a homeothermic blanket. Regional cerebral blood flow (rCBF) was monitored by Laser-Doppler flowmetry (FLOC1, OMEGAWAVE, Tokyo, Japan) to confirm whether the right hemisphere was ischemic or not. The surgery was considered successful when CBF was reduced by approximately 70-90% as described above. After 24 hours or 7 days of reperfusion, and after calculating the neurological scores using a grading system of neurological impairment, the mice were decapitated, and their brains were quickly removed and cooled to -30°C for 10 minutes. The brains were cut into five 2-mm-thick slices, incubated in 1% 2,3,5-triphenyltetrazolium chloride (TTC, Wako, Japan) for 20 min at 37°C, and immersed overnight in 4% paraformaldehyde (PFA; Sigma, USA). Non-infarcted areas were shown as gray, and infarcted areas were shown as white. Infarct volume was measured using ImageJ software and expressed as a percentage of the total hemisphere (corresponding to B in Fig. 19, E in Fig. 21, and H in Fig. 23. The vertical axis Neurological score in C of Fig. 19, F of Fig. 21, and I of Fig. 23 represents the neurological deficit. The vertical axis Survival rate (%) in Fig. 24 corresponds to the mouse survival rate).

The compound of Example 1 was suspended in 0.5% carboxymethyl cellulose (CMC), and the control group was

orally given an equivalent amount of 0.5% CMC.

The mice were subjected to tMCAO for 2 hours, and after 30 minutes of reperfusion, Example Compound 1 was administered at different concentrations (0.5, 0.1, 0.3, and 1.0 mg/kg). The results are shown in Fig. 18A and Figs. 19B and C. Fig. 18A and Figs. 19B and C are representative images of TTC staining (A), quantitative analysis of infarct volume (B), and neurological damage (C) at 24 hours after reperfusion (n = 7-9). Example Compound 1 (0.3 mg/kg) was administered at 0.5, 1, and 2 hours after reperfusion. The results are shown in Fig. 20D and Figs. 21E and F. Fig. 20D and Figs. 21E and F are representative images of TTC staining (D), quantitative analysis of infarct volume (E), and neurological damage (F) after 24 hours of reperfusion (n = 7-8). After 30 minutes of reperfusion, different doses (0.5, 0.1, and 0.3 mg/kg) of the Example 1 compound were administered. The results are shown in Fig. 22G and Figs. 23H and I. Fig. 22G and Figs. 23H and I are representative images of TTC staining (G), quantitative analysis of infarct volume (H), and neurological damage (I) at 7 days after reperfusion. The number of surviving mice per day in each group was recorded to calculate the survival rate (Fig. 24J). (n = 9-12). In particular, in mice given 0.3 mg/kg of Example Compound 1, cerebral infarct volume was reduced compared to Vehicle mice, and mouse survival rate was improved compared to Vehicle mice.

## Claims

**1.** A compound represented by formula (IX):

[Formula 1]

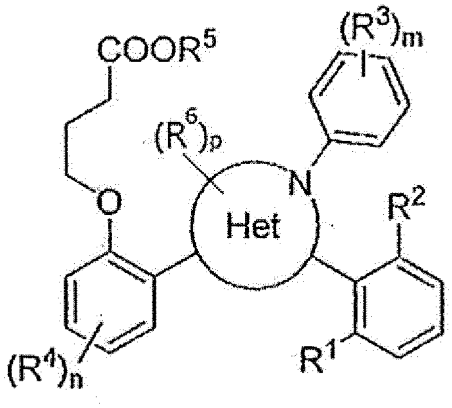

(IX)

wherein $R^1$ is a hydrogen atom or a $C_{1-6}$ haloalkyl;
$R^2$ is a $C_{1-6}$ haloalkyl;
$R^3$ is selected from hydroxys, $C_{1-6}$ alkyls, $C_{1-6}$ alkoxys and halogen atoms;
$R^4$ is selected from halogen atoms and $C_{1-6}$ alkyls;
$R^5$ is a hydrogen atom or a $C_{1-6}$ alkyl;
$R^6$ is a halogen atom or a $C_{1-6}$ alkyl;
m is an integer selected from 0 to 3;
n is an integer selected from 0 to 3;
p is an integer selected from 0 to 2; and
Het is a 5-membered nitrogen-containing aromatic heterocyclic ring with one nitrogen atom and two carbon atoms substituted with phenyl groups,

or a pharmaceutically acceptable salt thereof.

**2.** The compound or pharmaceutically acceptable salt thereof according to claim 1, wherein the compound is represented by formula (Ia):

[Formula 2]

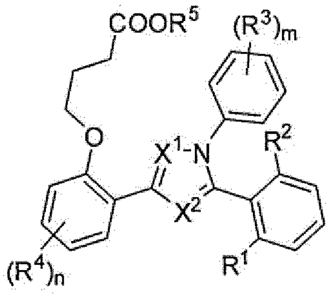

(I a)

wherein $R^1$, $R^2$, $R^3$, $R^4$, $R^5$, n, and m are as defined in claim 1;
$X^1$ and $X^2$ are independently a nitrogen atom or C-$R^{6a}$; and
$R^{6a}$ is a hydrogen atom, a halogen atom, or a $C_{1-6}$ alkyl.

**3.** The compound or pharmaceutically acceptable salt thereof according to claim 1 or claim 2, wherein $X^1$ is a nitrogen atom and $X^2$ is CH, or $X^1$ and $X^2$ are nitrogen atoms.

**4.** The compound or pharmaceutically acceptable salt thereof according to any one of claims 1 to 3, wherein $R^2$ is trifluoromethyl.

**5.** The compound or pharmaceutically acceptable salt thereof according to any one of claims 1 to 4, wherein m is 0 or 1 and n is 0 or 1.

**6.** The compound or pharmaceutically acceptable salt thereof according to any one of claims 1 to 4, wherein m is 0 and n is 1 or 0.

**7.** The compound or pharmaceutically acceptable salt thereof according to any one of claims 1 to 6, wherein the compound is represented by formula (Ib):

[Formula 3]

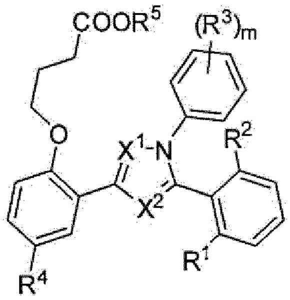

(I b)

wherein $R^1$, $R^2$, $R^3$, $R^4$, $R^5$, $X^1$, $X^2$, and m are as defined in any of claims 1 to 6.

**8.** The compound or pharmaceutically acceptable salt thereof according to any one of claims 1 to 7, wherein $R^1$ is a hydrogen atom.

**9.** The compound or pharmaceutically acceptable salt thereof according to claim 1, wherein the compound is selected from 4-(4-fluoro-2-(1-phenyl-5-(2-(trifluoromethyl)phenyl)-1H-pyrazol-3-yl)phenoxy)butanoic acid;

4-(2-(1-phenyl-5-(2-(trifluoromethyl)phenyl)-1H-pyrazol-3-yl)phenoxy)butanoic acid;
4-(2-(1-phenyl-5-(2-(trifluoromethyl)phenyl)-1H-1,2,4-triazol-3-yl)phenoxy)butanoic acid;
4-(4-fluoro-2-(1-(4-isopropylphenyl)-5-(2-(trifluoromethyl)phenyl)-1H-pyrazol-3-yl)phenoxy)butanoic acid; and
4-(2-(1-(4-isopropylphenyl)-5-(2-(trifluoromethyl)phenyl)-1H-pyrazol-3-yl)phenoxy)butanoic acid.

**10.** A pharmaceutical composition, comprising the compound or pharmaceutically acceptable salt thereof according to any one of claims 1 to 9.

**11.** The pharmaceutical composition according to claim 10, for use in the treatment or prevention of synucleinopathy.

**12.** The pharmaceutical composition according to claim 11, wherein the synucleinopathy is Parkinson's disease (PD), dementia with Lewy bodies (DLB), or multiple system atrophy.

**13.** The pharmaceutical composition according to claim 10, for use in the treatment or prevention of a disease caused by dopamine neuronal dysfunction.

**14.** The pharmaceutical composition according to claim 10, for use in the treatment or prevention of a cerebrovascular disorder such as cerebral infarction, cerebral hemorrhage, and subarachnoid hemorrhage, and head injury.

**15.** The pharmaceutical composition according to claim 10, for use in the treatment or prevention of a psychiatric disorder such as autism and schizophrenia.

**16.** The pharmaceutical composition according to any one of claims 10 to 15, which is for oral administration.

# Fig. 1

>FABP3

atggcggacgccttcctgggcacctggaagctagtggacagcaagaatttcgatgactacatgaagtcactcggtgtggg
ttttgctaccaggcaggtggccagcatgaccaagcctaccacaatcatcgaaaagaatggggacattctcaccctaaaaa
cacacagcaccttcaagaacacagagatcagctttaagttgggggtggagttcgatgagacaacagcagatgacaggaag
gtcaagtccattgtgacactggatggagggaaacttgttcacctgcagaaatgggacgggcaagagaccacacttgtgcg
ggagctaattgatggaaaactcatcctgacactcacccacggcactgcagtttgcactcgcacttatgagaaagaggctt
aa

>FABP4

atgtgtgatgcttttgtaggtacctggaaacttgtctccagtgaaaactttgatgattatatgaaagaagtaggagtggg
ctttgccaccaggaaagtggctggcatggccaaacctaacatgatcatcagtgtgaatggggatgtgatcaccattaaat
ctgaaagtacctttaaaaatactgagatttccttcatactgggccaggaatttgacgaagtcactgcagatgacaggaaa
gtcaagagcaccataaccttagatggggtgtcctggtacatgtgcagaaatgggatggaaaatcaaccaccataaagag
aaaacgagaggatgataaactggtggtggaatgcgtcatgaaaggcgtcacttccacgagagtttatgagagagcat

>FABP5

atggccacagttcagcagctggaaggaagatggcgcctggtggacagcaaaggctttgatgaatacatgaaggagctagg
agtgggaatagctttgcgaaaaatgggcgcaatggccaagccagattgtatcatcacttgtgatggtaaaaacctcacca
taaaaactgagagcactttgaaaacaacacagttttcttgtaccctgggagagaagtttgaagaaaccacagctgatggc
agaaaaactcagactgtctgcaactttacagatggtgcattggttcagcatcaggagtgggatgggaaggaaagcacaat
aacaagaaaattgaaagatgggaaattagtggtggagtgtgtcatgaacaatgtcacctgtactcggatctatgaaaaag
tagaataa

>FABP7

atggtggaggctttctgtgctacctggaagctgaccaacagtcagaactttgatgagtacatgaaggctctaggcgtggg
ctttgccactaggcaggtgggaaatgtgaccaaaccaacggtaattatcagtcaagaaggagacaaagtggtcatcagga
ctctcagcacattcaagaacacggagattagtttccagctgggagaagagtttgatgaaaccactgcagatgatagaaac
tgtaagtctgttgttagcctggatggagacaaacttgttcacatacagaaatgggatggcaaagaaacaaattttgtaag
agaaattagggatggcaaaatggttatgacccttacttttggtgatgtggttgctgttcgccactatgagaaggcttaa

Fig. 2

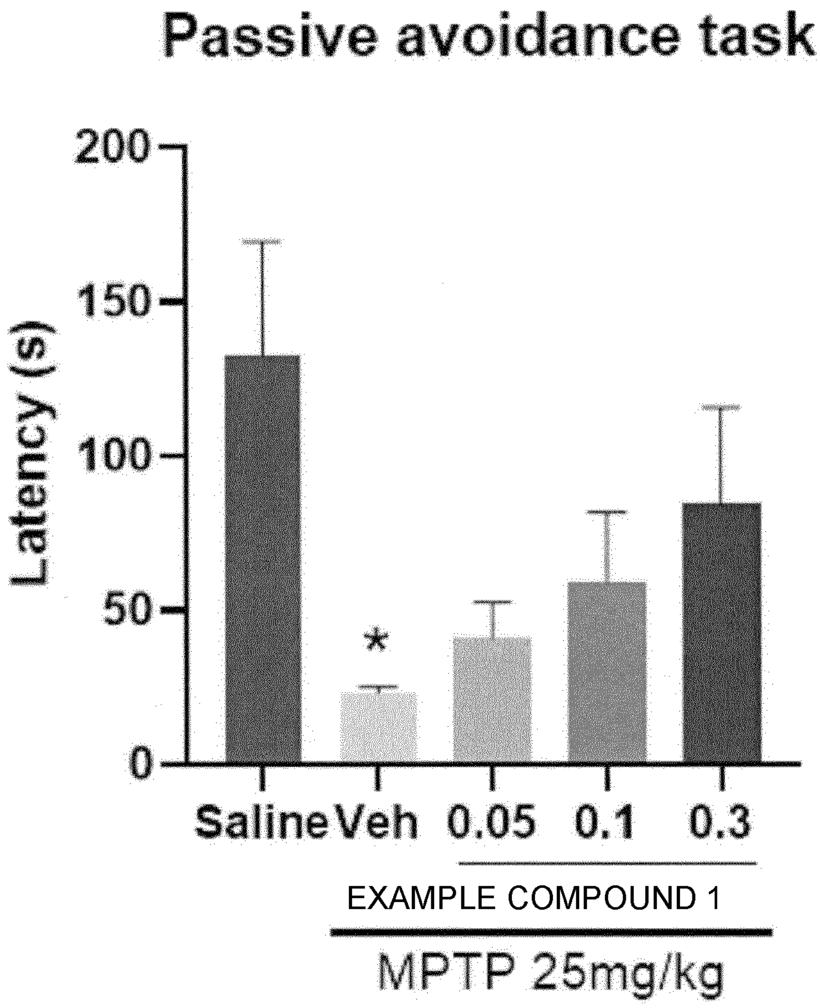


* p < 0.05 vs. Saline.

Fig. 3

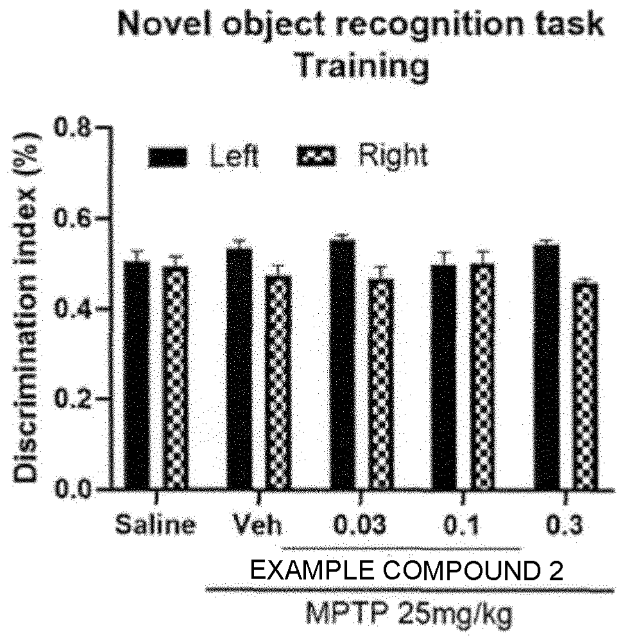


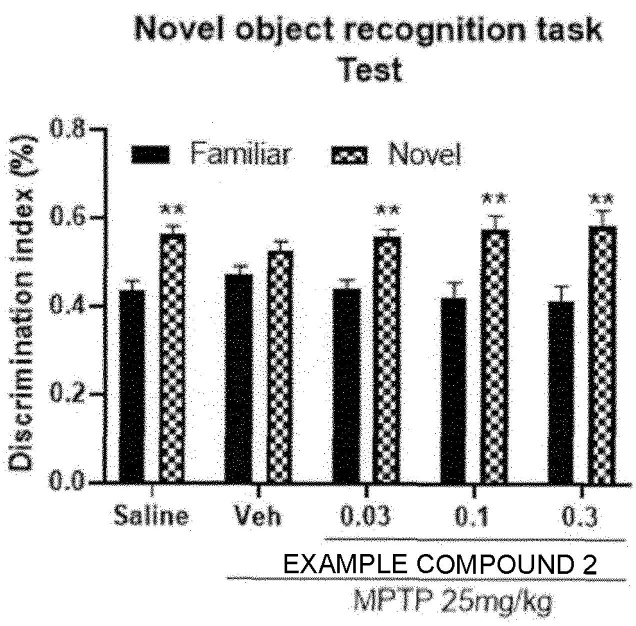


** $p < 0.01$ vs. Familiar.

Fig. 4

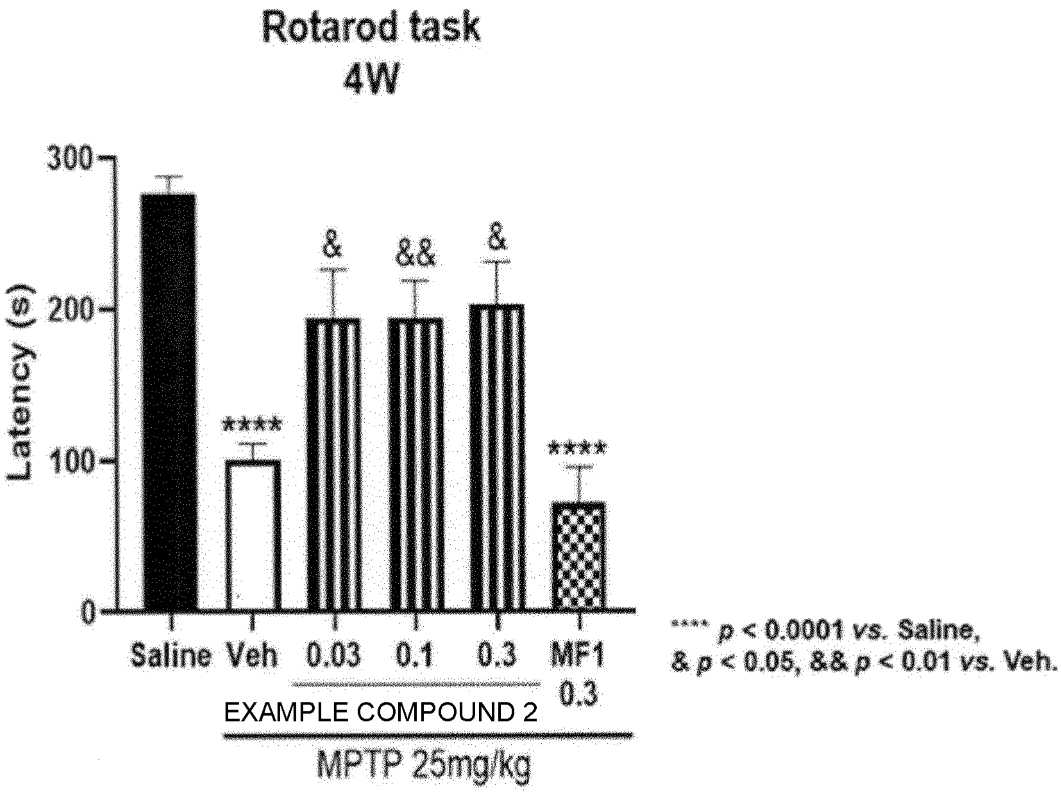
Rotarod task
4W
Latency (s)
300
200
100
0
&
&&
&
****
****
Saline
Veh
0.03
0.1
0.3
MF1
0.3
EXAMPLE COMPOUND 2
MPTP 25mg/kg
**** p < 0.0001 vs. Saline,
& p < 0.05, && p < 0.01 vs. Veh.

Fig. 5

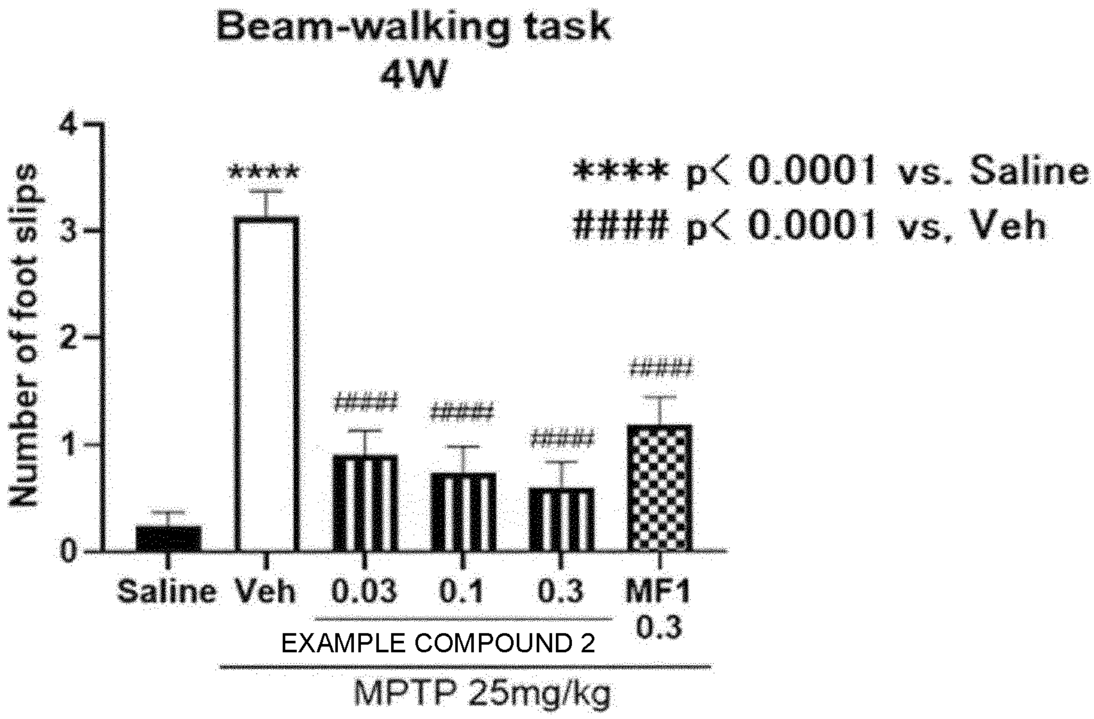
Beam-walking task
4W
Number of foot slips
4
3
2
1
0
****
####
####
####
####
Saline
Veh
0.03
0.1
0.3
MF1
0.3
EXAMPLE COMPOUND 2
MPTP 25mg/kg
**** p< 0.0001 vs. Saline
#### p< 0.0001 vs. Veh

Fig. 6

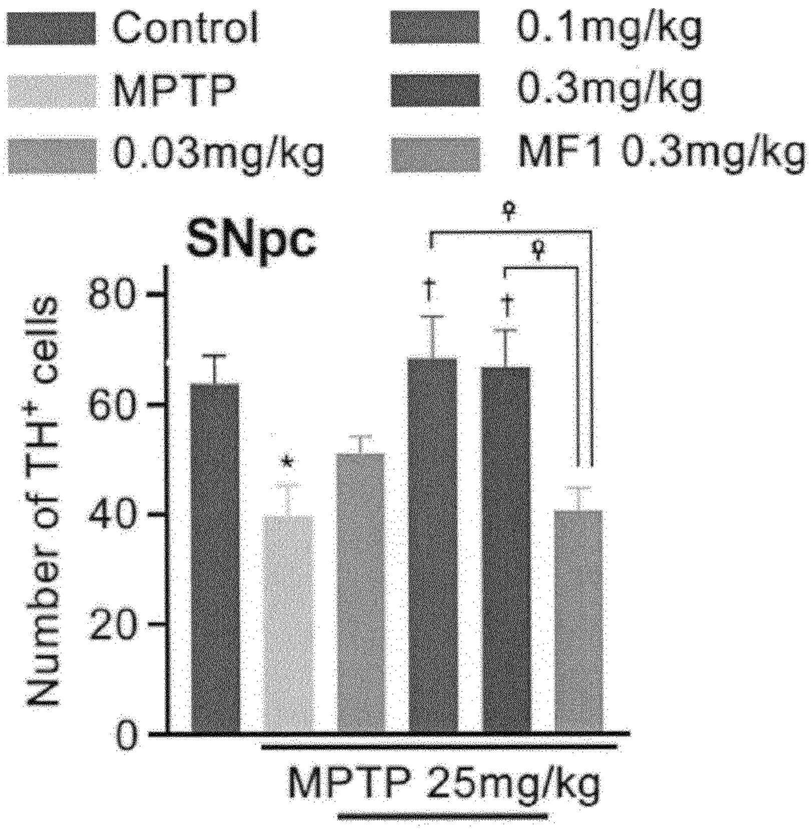


EXAMPLE COMPOUND 2

Error bars represent SEM. *$p < 0.05$ vs. vehicle-treated saline mice; †$p < 0.05$ vs. vehicle-treated MPTP mice; ♀$p < 0.05$ vs. MF-1 (0.3 mg/kg)-treated group.

Fig. 7

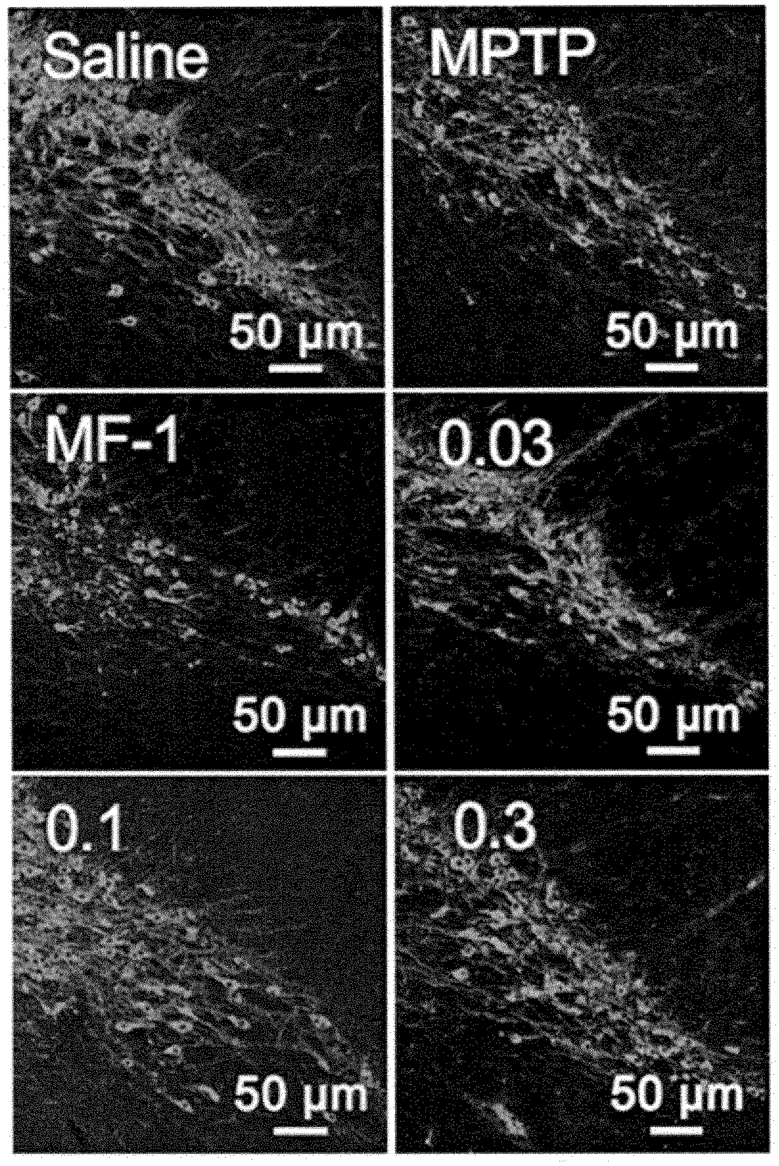

Fig. 8

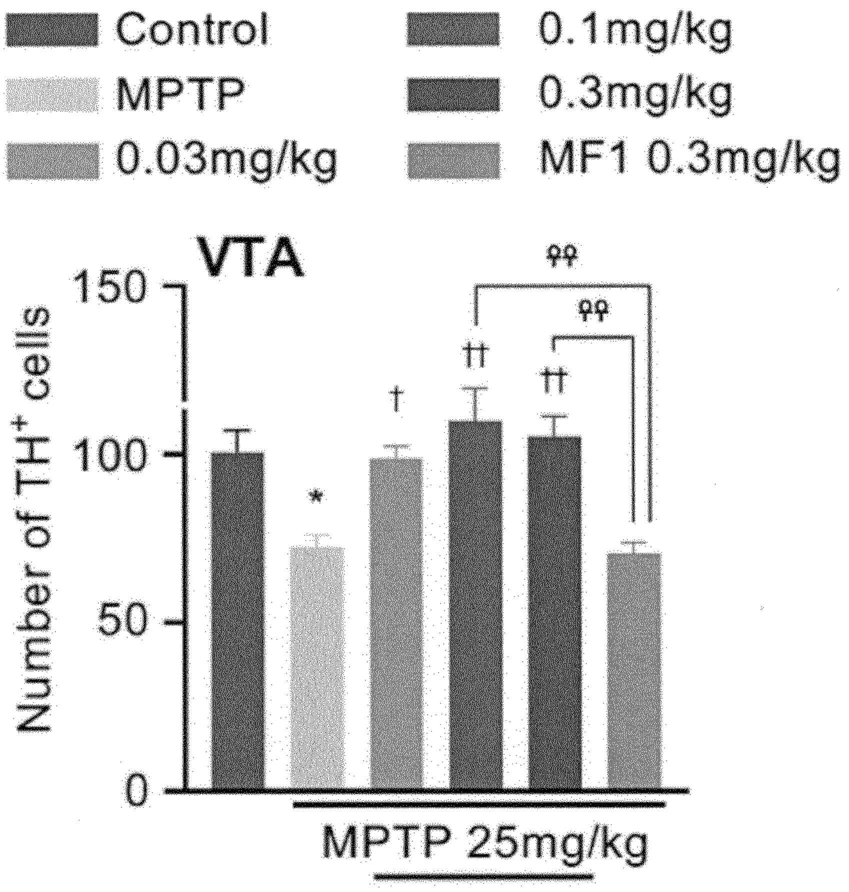


EXAMPLE COMPOUND 2

Error bars represent SEM. *$p < 0.05$ vs. vehicle-treated saline mice; †$p < 0.05$, and ††$p < 0.01$ vs. vehicle-treated MPTP mice; ♀♀$p < 0.01$ vs. MF-1 (0.3 mg/kg)-treated group.

Fig. 9

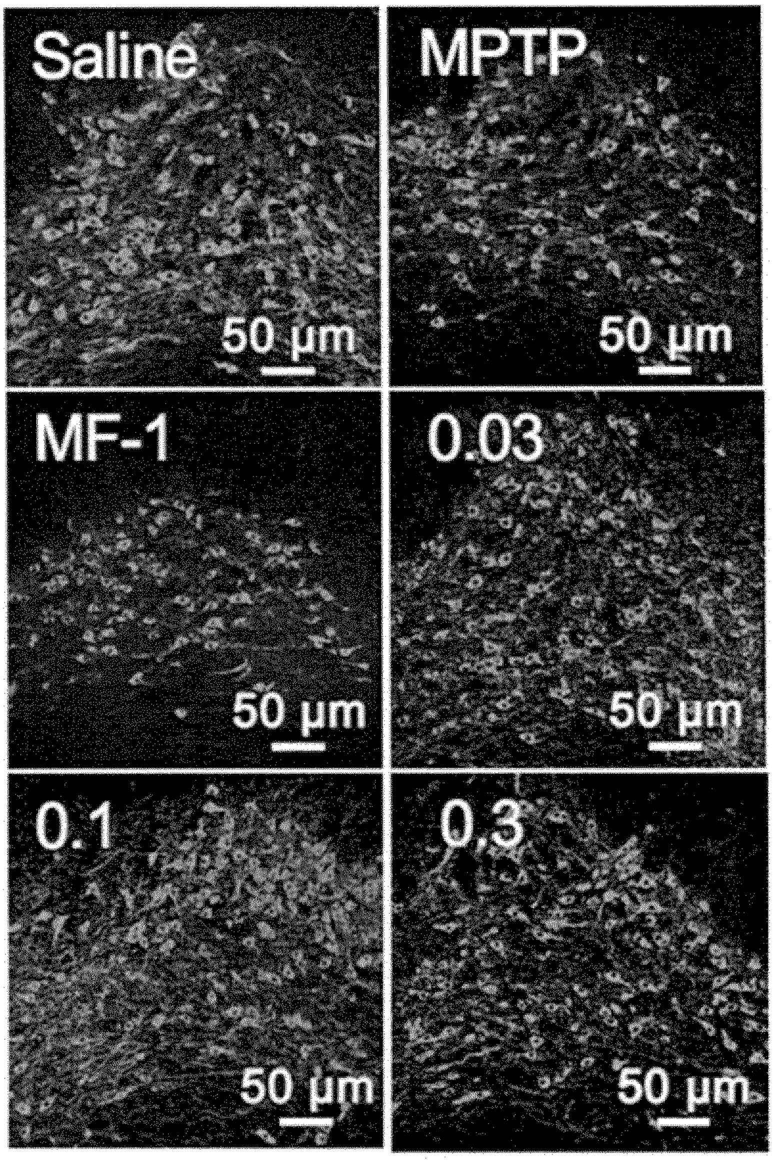

Fig. 10

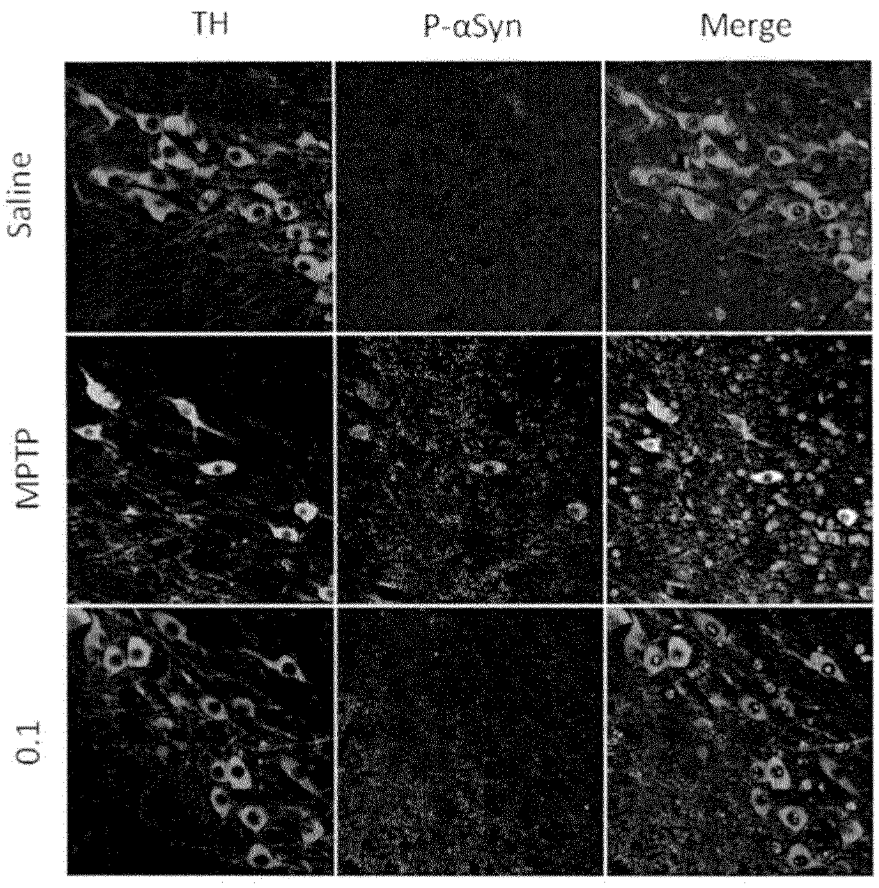


Fig. 11

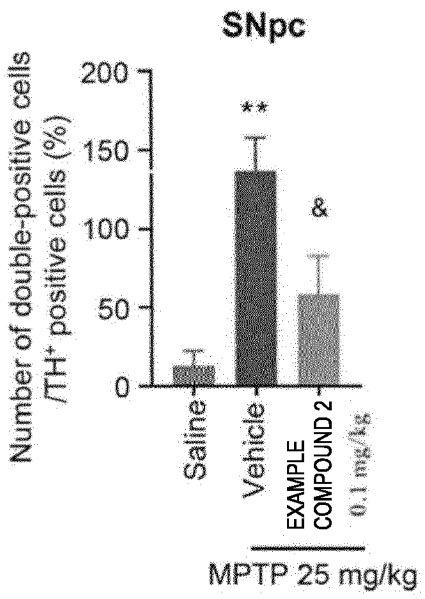


Error bars represent SEM. *p < 0.05, and **p < 0.01 vs. vehicle-treated saline mice; &p < 0.05 vs. vehicle-treated MPTP mice.

Fig. 12

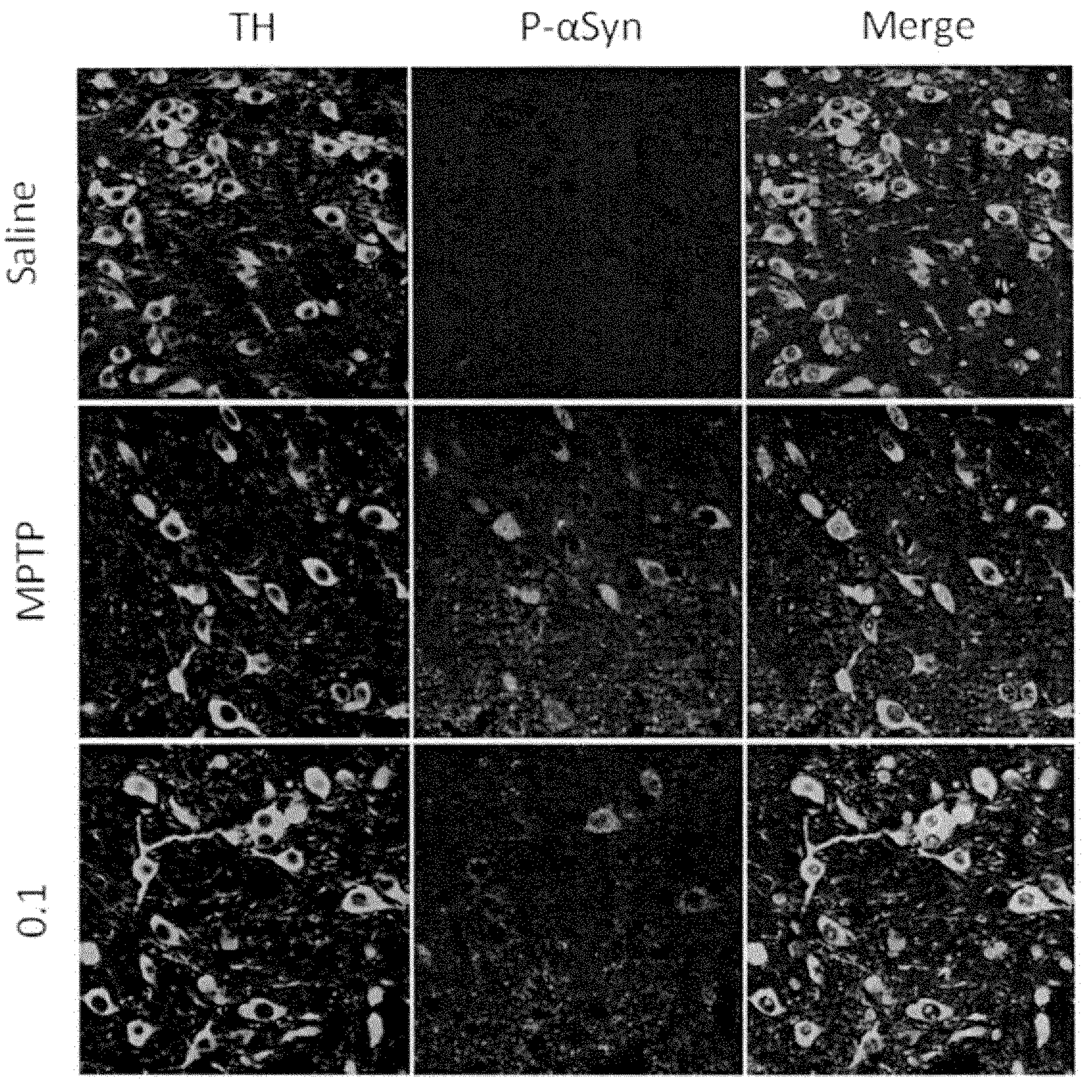

Fig. 13

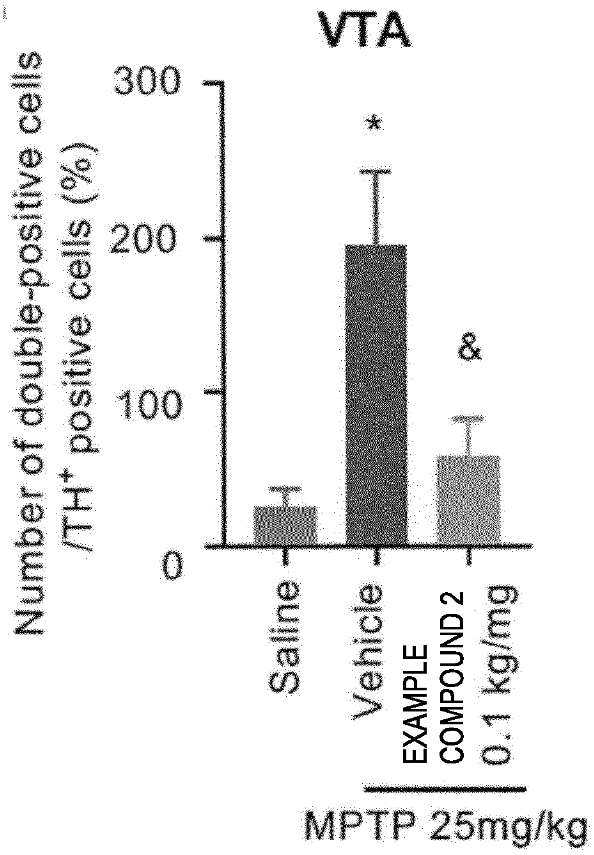


Error bars represent SEM. *p < 0.05 vs. vehicle-treated saline mice; &p < 0.05 vs. vehicle-treated MPTP mice.

Fig. 14

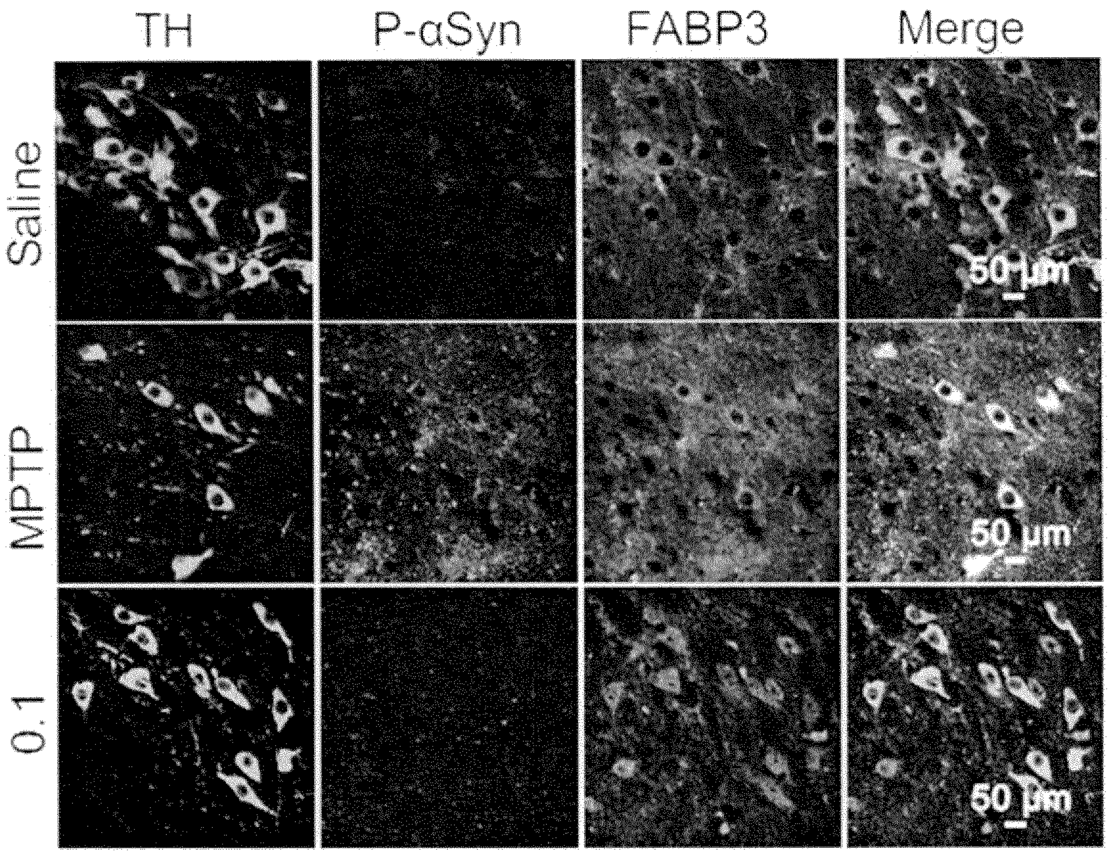

Fig. 15

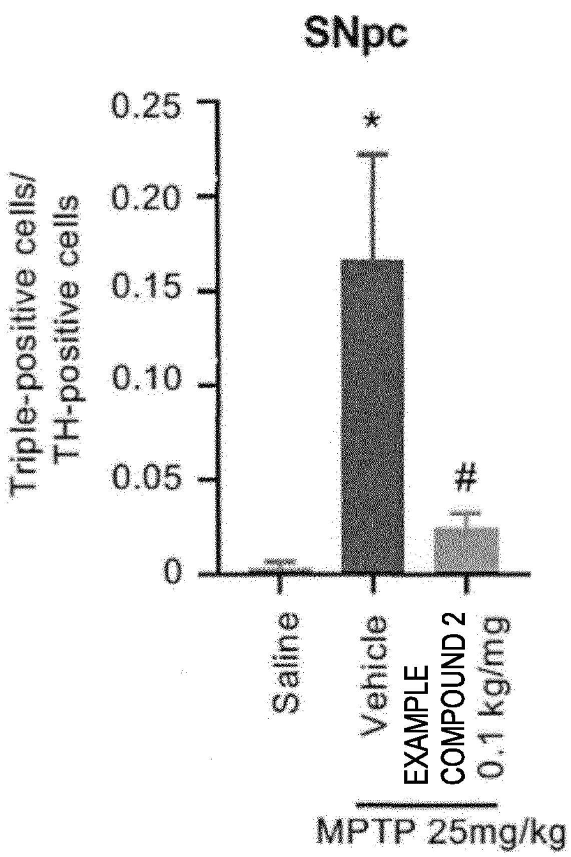


Error bars represent SEM. *p < 0.05 vs. vehicle-treated saline mice; #p < 0.05 vs. vehicle-treated MPTP mice.

Fig. 16

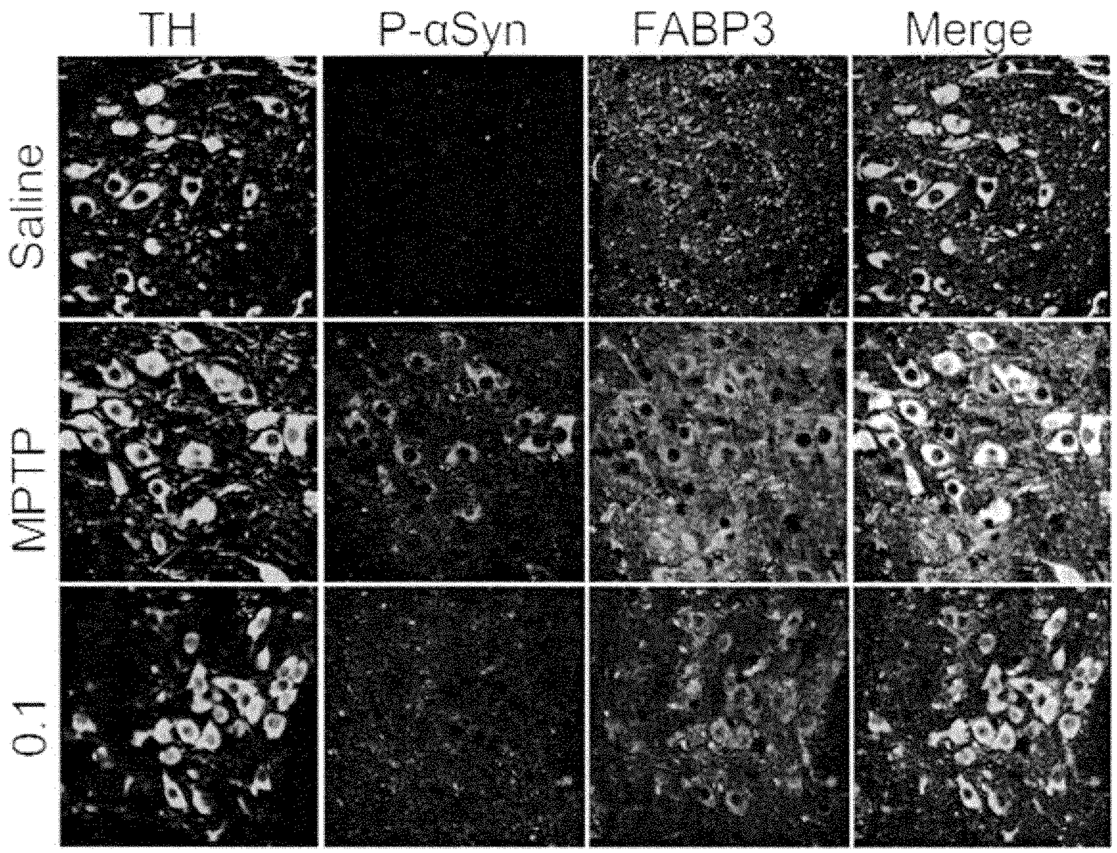

Fig. 17

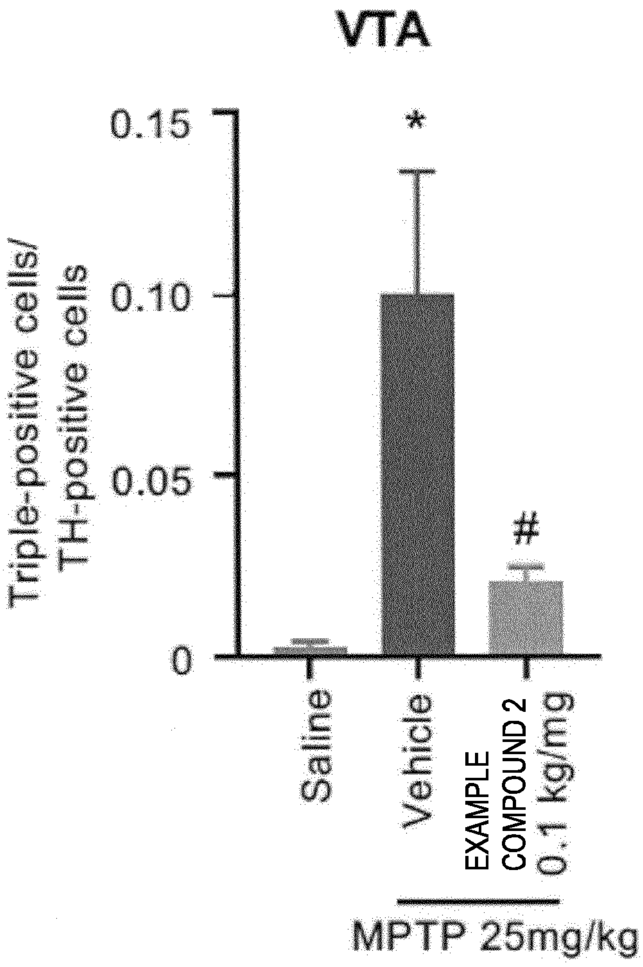


Error bars represent SEM. *p < 0.05 vs. vehicle-treated saline mice; #p < 0.05 vs. vehicle-treated MPTP mice.

Fig. 18

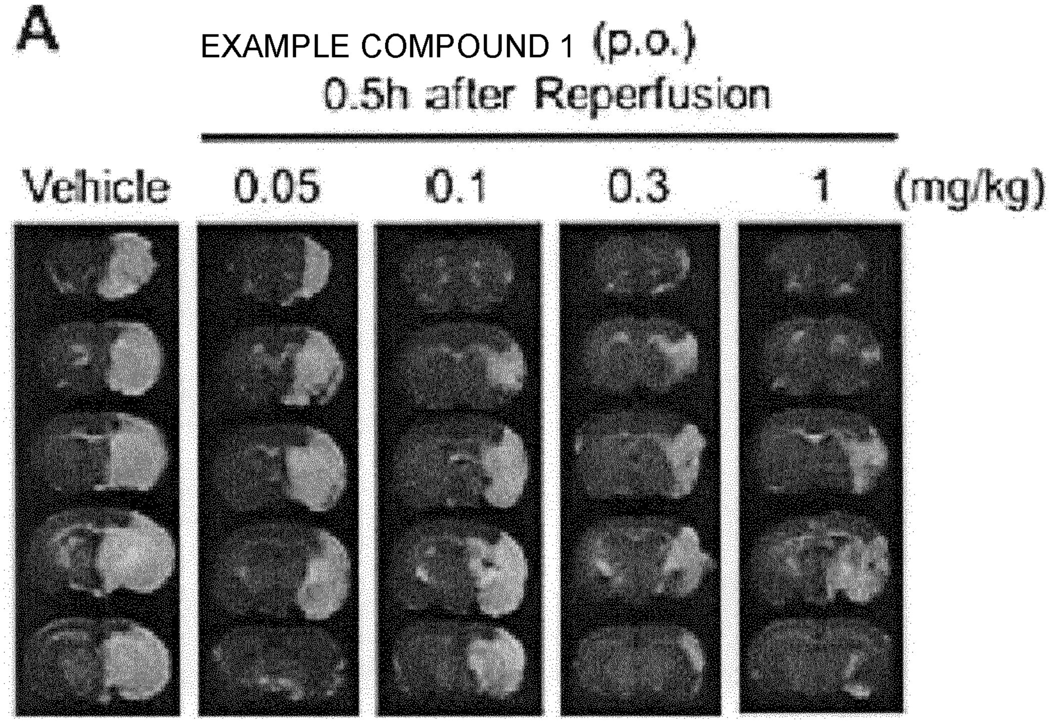
A
EXAMPLE COMPOUND 1 (p.o.)
0.5h after Reperfusion
Vehicle
0.05
0.1
0.3
1
(mg/kg)

Fig. 19

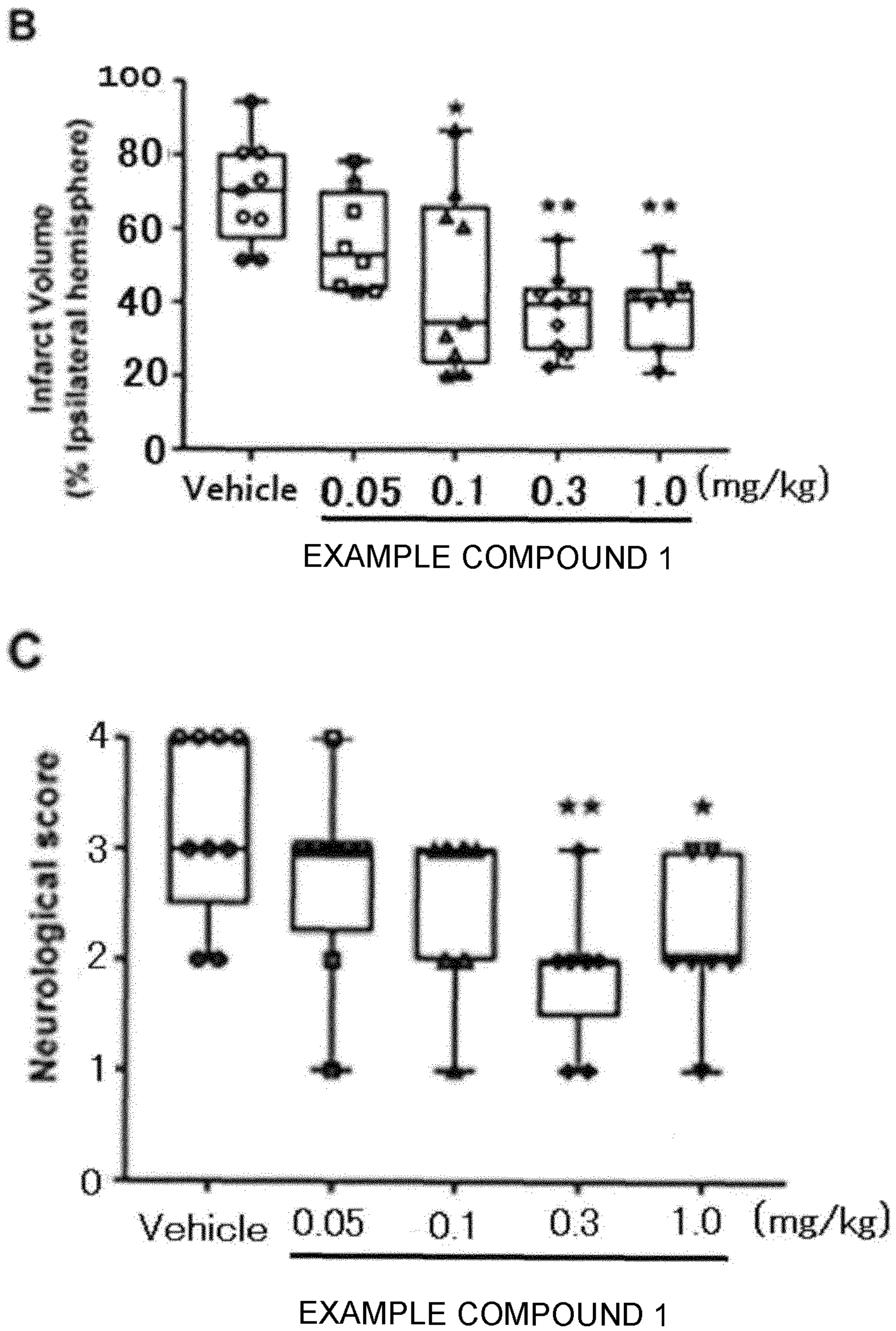


The data shown in each column represent the mean ± SEM. *$p < 0.05$, **$p < 0.01$ vs. Vehicle-treated I/R group.

Fig. 20

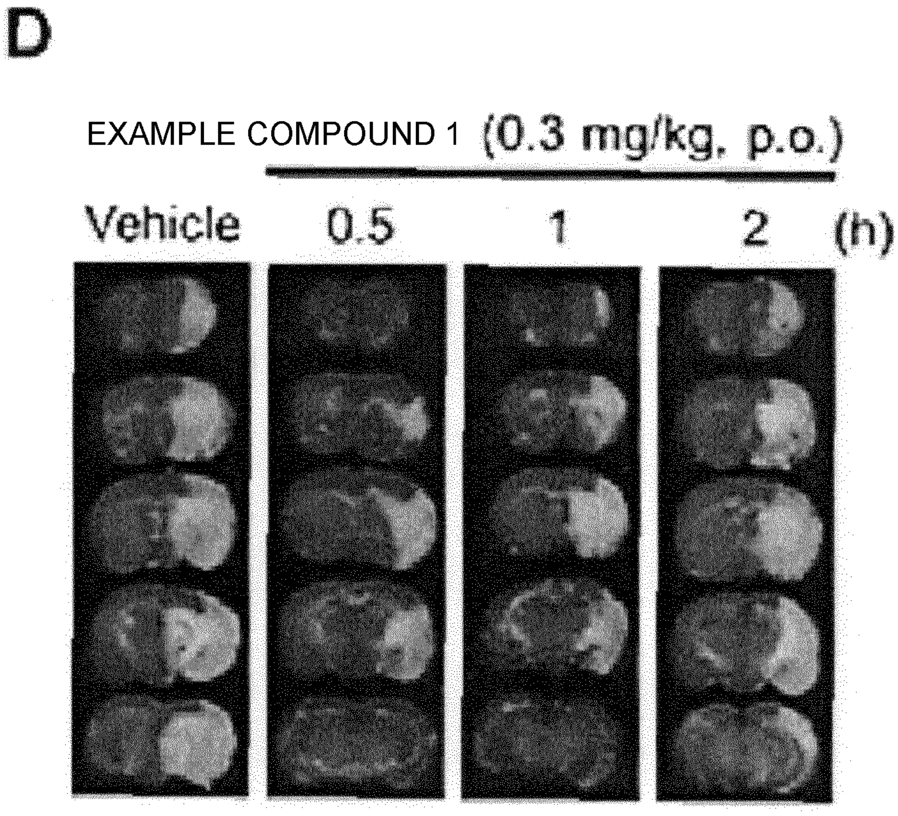

Fig. 21

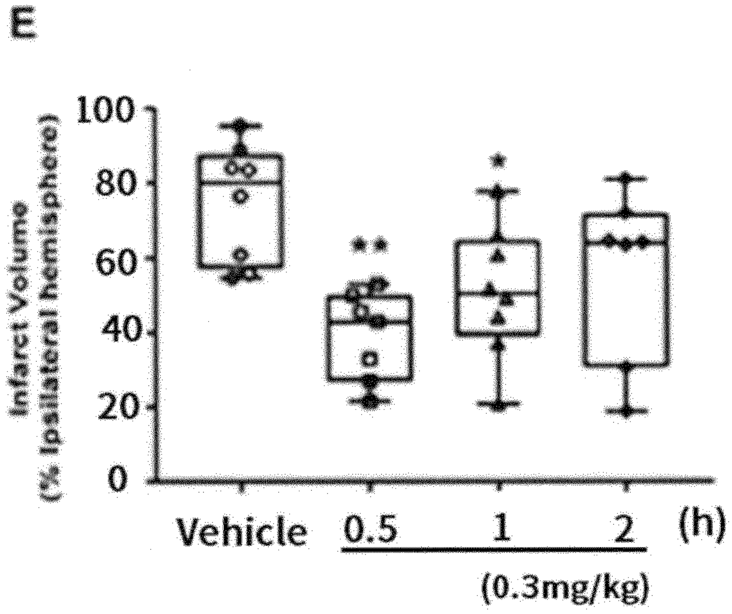


EXAMPLE COMPOUND 1

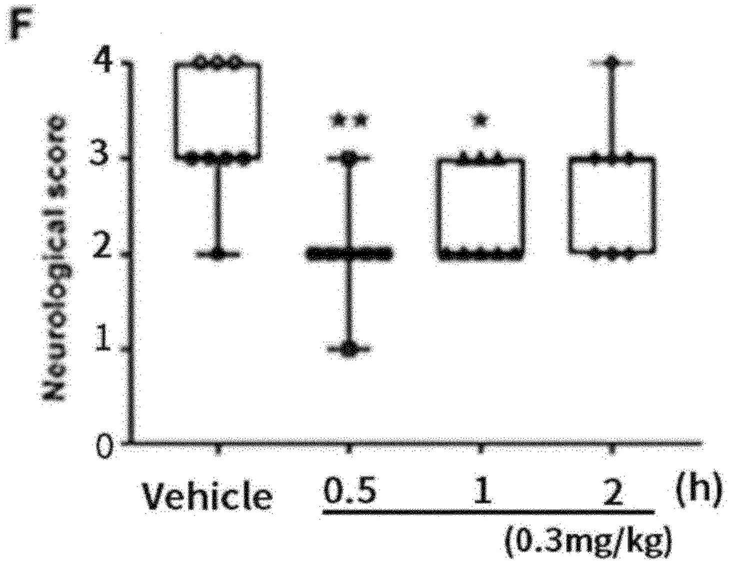


EXAMPLE COMPOUND 1

The data shown in each column represent the mean ± SEM. *p < 0.05, **p < 0.01 vs. Vehicle-treated I/R group.

Fig. 22

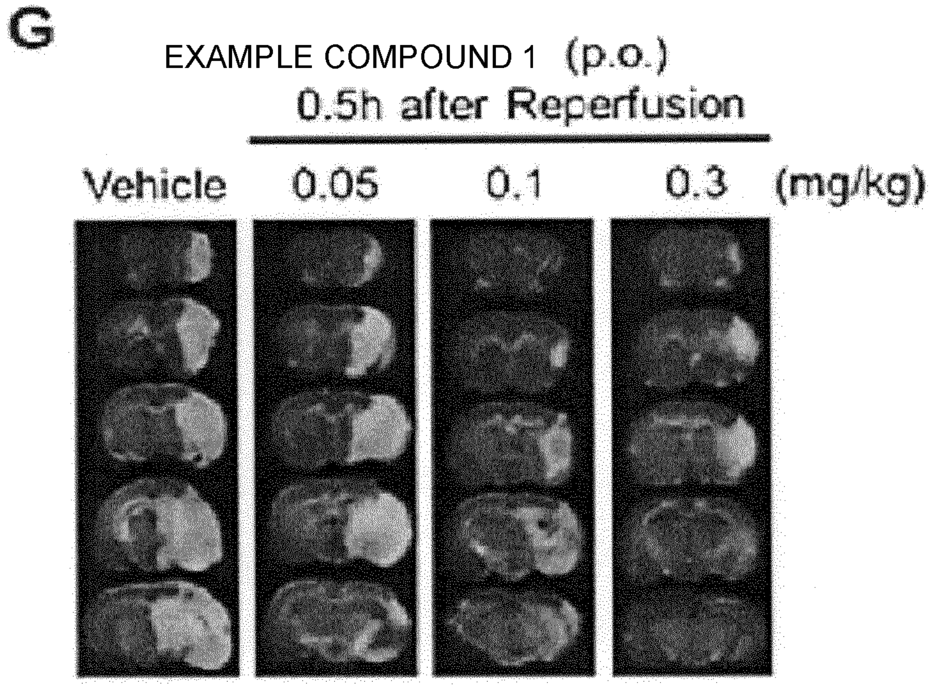
G
EXAMPLE COMPOUND 1 (p.o.)
0.5h after Reperfusion
Vehicle
0.05
0.1
0.3
(mg/kg)

Fig. 23

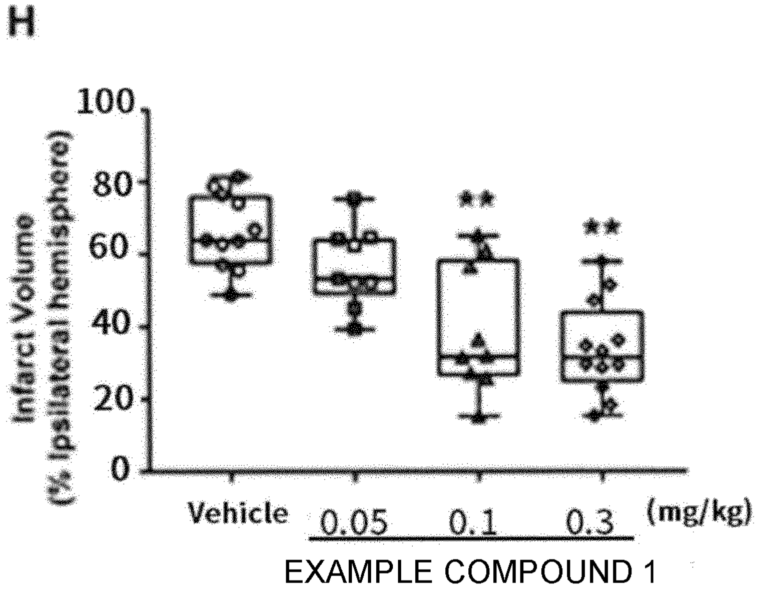


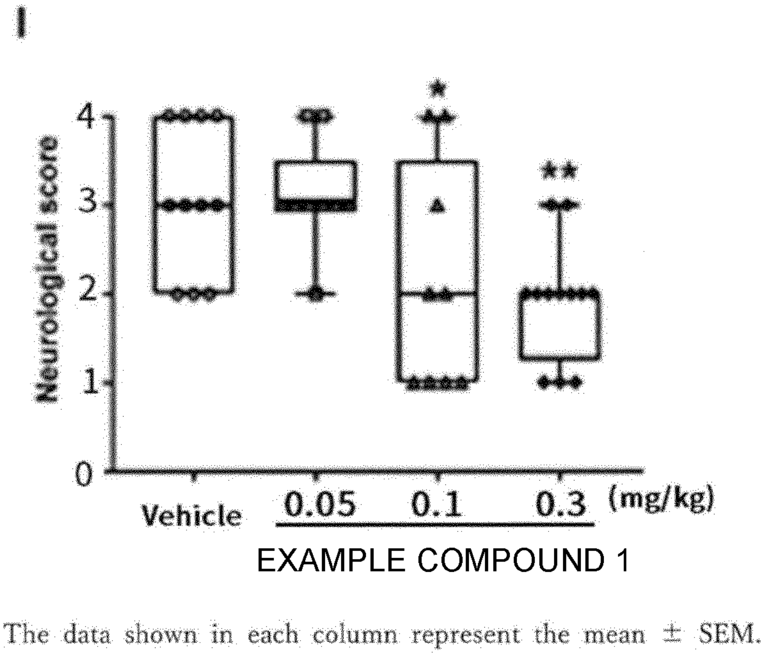


The data shown in each column represent the mean ± SEM. *p < 0.05, **p < 0.01 vs. Vehicle-treated I/R group.

Fig. 24

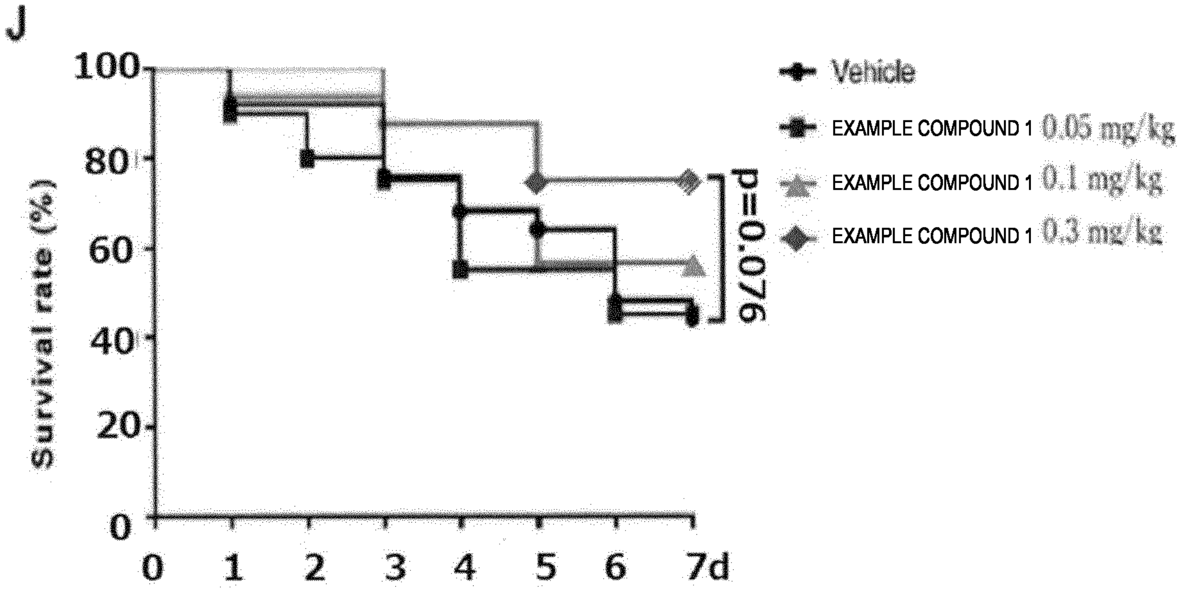
J
100
80
60
40
20
0
Survival rate (%)
0
1
2
3
4
5
6
7d
p=0.076
Vehicle
EXAMPLE COMPOUND 1 0.05 mg/kg
EXAMPLE COMPOUND 1 0.1 mg/kg
EXAMPLE COMPOUND 1 0.3 mg/kg

Fig. 25

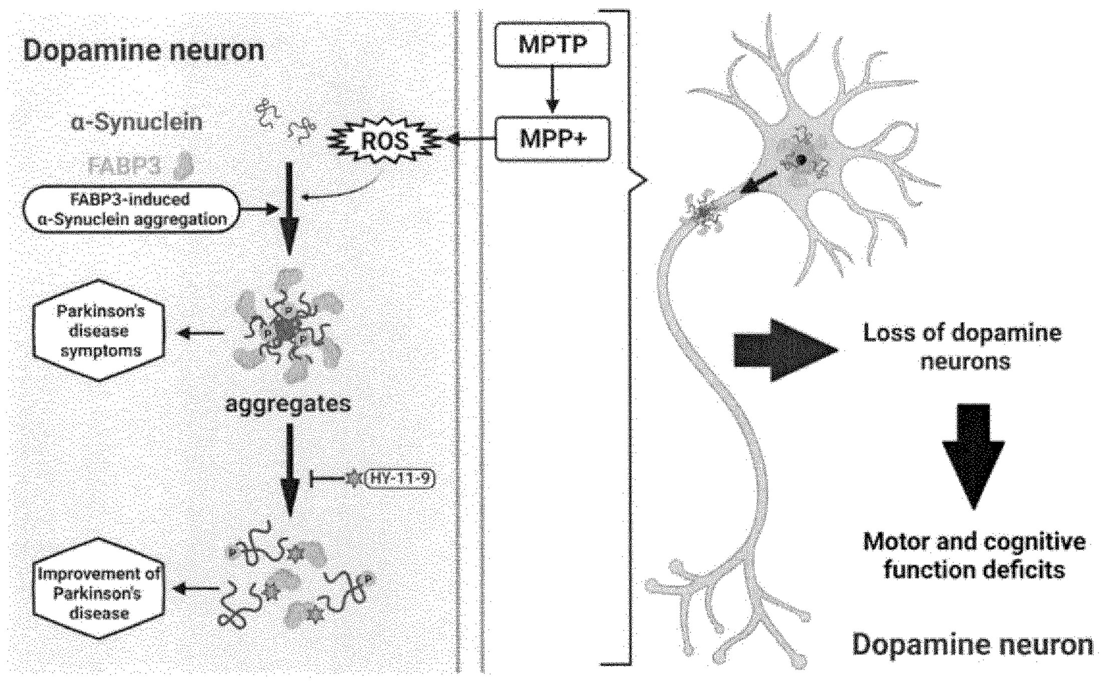
Dopamine neuron
α-Synuclein
FABP3
ROS
MPTP
MPP+
FABP3-induced
α-Synuclein aggregation
Parkinson's
disease
symptoms
aggregates
HY-11-9
Improvement of
Parkinson's
disease
Loss of dopamine
neurons
Motor and cognitive
function deficits
Dopamine neuron

## INTERNATIONAL SEARCH REPORT

International application No.
**PCT/JP2023/034339**

**A. CLASSIFICATION OF SUBJECT MATTER**

***C07D 231/12***(2006.01)i; ***A61K 31/415***(2006.01)i; ***A61K 31/4196***(2006.01)i; ***A61P 9/10***(2006.01)i; ***A61P 17/02***(2006.01)i; ***A61P 25/00***(2006.01)i; ***A61P 25/02***(2006.01)i; ***A61P 25/16***(2006.01)i; ***A61P 25/28***(2006.01)i; ***C07D 249/08***(2006.01)i; ***C12N 15/12***(2006.01)i
FI: C07D231/12 C CSP; A61K31/4196; A61P9/10; A61P25/00; A61P25/02; A61P25/16; A61P25/28; A61P17/02; C07D249/08 535; C12N15/12; A61K31/415 ZNA

According to International Patent Classification (IPC) or to both national classification and IPC

**B. FIELDS SEARCHED**

Minimum documentation searched (classification system followed by classification symbols)

C07D231/12; A61K31/415; A61K31/4196; A61P9/10; A61P17/02; A61P25/00; A61P25/02; A61P25/16; A61P25/28; C07D249/08; C12N15/12

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| A | WO 2017/171053 A1 (TOHOKU UNIV.) 05 October 2017 (2017-10-05)<br>claims, paragraphs [0001]-[0122], examples | 1-16 |
| A | BENIYAMA, Yoko et al. Structure-guided design, synthesis and in vitro evaluation of a series of pyrazole-based fatty acid binding protein (FABP) 3 ligands. Bioorganic & Medicinal Chemistry Letters. 2013, 23, 1662-1666, DOI:10.1016/j.bmcl.2013.01.054<br>column of abstract, body text | 1-16 |
| A | SHINODA, Yasuharu et al. Analysis of binding affinity and docking of novel fatty acid-binding protein (FABP) ligands. Journal of Pharmacological Sciences. 2020, 143, 264-271, DOI:10.1016/j.jphs.2020.05.005<br>columns of 1. Introduction, 2. Materials and Methods, 4. Discussion | 1-16 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **16 November 2023** | **05 December 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | <br>Telephone No. |

Form PCT/ISA/210 (second sheet) (January 2015)

INTERNATIONAL SEARCH REPORT

International application No.
PCT/JP2023/034339

C. DOCUMENTS CONSIDERED TO BE RELEVANT

| Category* | Citation of document, with indication, where appropriate, of the relevant passages | Relevant to claim No. |
|---|---|---|
| A | TAN, M. C. et al. Interaction kinetics of liposome-incorporated unsaturated fatty acids with fatty acid-binding protein 3 by surface plasmon resonance. Bioorganic & Medicinal Chemistry. 22, 2014, 1804-1808, DOI:10.1016/j.bmc.2014.02.001<br>columns of abstract, 3. Results and discussion | 1-16 |

Form PCT/ISA/210 (second sheet) (January 2015)

**INTERNATIONAL SEARCH REPORT**

International application No.
**PCT/JP2023/034339**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      - ☑ in the form of an Annex C/ST.25 text file.
      - ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      - ☐ in the form of an Annex C/ST.25 text file (Rule 13*ter*.1(a)).
      - ☐ on paper or in the form of an image file (Rule 13*ter*.1(b) and Administrative Instructions, Section 713).
2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.
3. Additional comments:

   "In the form of an Annex C/ST.25 text file" above should be understood as "in ST.26 format".

Form PCT/ISA/210 (continuation of first sheet) (January 2015)

**INTERNATIONAL SEARCH REPORT**
**Information on patent family members**

International application No.
**PCT/JP2023/034339**

| Patent document cited in search report | Publication date (day/month/year) | Patent family member(s) | Publication date (day/month/year) |
|---|---|---|---|
| WO 2017/171053 A1 | 05 October 2017 | US 2019/0125726 A1<br>claims, paragraphs [0001]-[0218], examples<br>EP 3437640 A1 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2017171053 A


### Non-patent literature cited in the description


- **SHARON, R et al.** *Neuron*, 20 February 2003, vol. 37, 583-595
- **YAMAMOTO, Y. et al.** *J. Neurosci.*, 2018, vol. 38 (49), 10411-10423
- **PAUMIER, K. L. et al.** *Neurobiology of Disease*, 2015, vol. 82, 185-199
- **MISAKI ONOSATO et al.** *52nd Conference of the Pharmaceutical Society of Japan Tohoku Branch Abstracts*, 2013, vol. 52, 47
- **SHIODA, N. et al.** *J. Biol. Chem.*, 2014, vol. 289, 18957-18965
- **MATSUO, K. et al.** *Neuropharmacology*, 2019, vol. 150, 164-174
- *Journal of Pharmacological Sciences*, 2023, vol. 152, 30-38
- **T.W. GREENE** ; **P.G.M. WUTS**. Protective Groups in Organic Synthesis. John Wiley and Sons, inc., 1999
- **SAMUEL, F. et al.** *J. Biol. Chem.*, 2016, vol. 291, 4374-4385
- **FUKUI, N. et al.** *J. Biol. Chem.*, 2021, vol. 296, 100663
- **LEE, V. M. Y. et al.** *J. Exp. Med.*, vol. 209 (5), 975-986
- **LI, Y-M. et al.** *Sci Rep.*, 2016, vol. 6, 37130